# EUROPEAN PATENT APPLICATION

(11) **EP 4 807 016 A1**
(43) Date of publication of application: **16.09.2026**
(21) Application number: 25163460.6
(22) Date of filing: 13.03.2025
(51) Int. Cl.: C12Q 1/6818, C12Q 1/6876

(54) **REPORTER PROBE FOR NUCLEIC ACID DETECTION**

(71) Applicant: Universitätsklinikum Hamburg-Eppendorf (UKE), 20246 Hamburg (DE)
(72) Inventor: Nörz, Dominik Sebastian, 20246 Hamburg (DE); Lütgehetmann, Marc, 20246 Hamburg (DE); Aepfelbacher, Martin, 20246 Hamburg (DE)
(74) Representative: Stüven, Ralf

(57) **Abstract**

The invention relates to a novel reporter probe for the detection and/or amplification of a nucleic acid. The reporter probe comprises a head and a tail oligonucleotide being configured to anneal to adjacent target regions on a target nucleic acid and to each other. The tail oligonucleotide is dual-labeled with a quencher and a fluorophore in a portion of the tail oligonucleotide branching from the target nucleic acid when bound to the target nucleic acid. The reporter probe can, for example, be used in single- or multiplex PCR or isothermal amplification methods.

## Description

The invention relates to a reporter probe for the detection and/or amplification of a nucleic acid, e.g. DNA. Further, the invention relates to uses of the reporter probe, to methods for the detection and/or amplification of a nucleic acid, and to a kit comprising the reporter probe.

Nucleic acid analysis is of great and growing importance, for example, in the context of diagnosis of infectious diseases. A variety of different methods for the detection and/or quantification and/or the amplification of nucleic acids in a biological sample have been developed and are in use today. Although there are amplification-free detection methods, e.g. CRISPR/Cas-based detection methods, many nucleic acid tests (NATs), are based on the amplication of nucleic acids. A well-established nucleic acid amplification tests (NAATs) is the polymerase chain reaction (PCR). While PCR depends on the use of thermocyclers because of the need to perform the different method amplification steps (denaturation, annealing, and extension) at different temperatures, isothermal amplification methods (INAATs) can be performed at a constant temperature. Examples of INAATs are Nucleic Acid Sequence-based Amplification (NASBA), Loop-mediated Isothermal Amplification (LAMP), Strand Displacement Amplification (SDA), Recombinase Polymerase Amplification (RPA) and Rolling Circle Amplification (RCA) (see, e.g., Oliveira BB, Veigas B and Baptista PV (2021) Isothermal Amplification of Nucleic Acids: The Race for the Next "Gold Standard", Front. Sens. 2:752600, doi: 10.3389/fsens.2021.752600; Zhao Y, Chen F, Li Q, Wang L, Fan C. Isothermal Amplification of Nucleic Acids. Chem Rev. 2015 Nov 25;115(22):12491-545. doi: 10.1021/acs.chemrev.5b00428. Epub 2015 Nov 9. PMID: 26551336).

The polymerase chain reaction (PCR) has, for example, been used routinely in clinical infection diagnostics for over 20 years. This method is based on the amplification of target DNA (DNA templates) in clinical samples by using a thermostable DNA polymerase and specific DNA primers or, in the case of target RNA (e.g. RNA viruses), by prior reverse transcriptase reaction followed by cDNA synthesis. The DNA can be detected during or after the amplification reaction, for example by gel electrophoresis, double-stranded DNA dyes, hybridization probes or so-called Taqman probes. Taqman assays, which are currently used particularly frequently in infection diagnostics, are based on the use of Taq polymerase with 5' exonuclease activity and double fluorescently labeled oligonucleotides (consisting of fluorophore and quencher) as Taqman probes. During amplification, the degradation of the Taqman probe leads to the release and thus dequenching of the fluorophore and its signal can be detected in real time with a real-time thermocycler. By using different fluorophore/quencher pairs and corresponding detector filter sets in thermocyclers, up to five (rarely up to seven) different parameters/target sequences can be detected in one reaction (multiplex PCR). This enables the implementation of full process controls and the simultaneous detection of jointly sought target nucleic acids (e.g. viruses, bacteria and parasites). Such multiplex procedures can significantly increase diagnostic efficiency due to their lower use of materials and personnel. In recent years, also due to these technical developments, there has been a strong increase in indication-based pathogen panel diagnostics (syndromic panels) using multiplex procedures. Sometimes more than 20 different parameters are tested and differentiated.

In order to use the multiplexing potential of PCR on commercial real-time thermocyclers, different processes and systems have been proposed and are commercially availabe. In one such process called "TOCE" ("Tagging Oligonucleotide Cleavage and Extension"; Seegene Inc., South Korea; WO 2014/104818 A1; Lee D-H, 2012, Seegene Bulletin 1, 5-10, available under https://seegenebrazil.com.br/wp-content/uploads/2019/07/TOCE_Seegene-ArtigoCientifico.pdf) the classic Taqman probe is replaced by an oligonucleotide (called "pitcher") having a targeting portion binding to a target sequence and a tagging portion at the 5' end of the probe sequence instead of a fluorescent label. After digestion of the "pitcher" oligo, the tag sequence is released and acts as a primer that binds to a second, synthetic template oligo (called "catcher"). This "catcher" oligo, which does not bind to the target sequence, is labeled with a fluorophore and quencher (distance approx. 21 bases). After binding of the "pitcher" primer, a counterstrand to the "catcher" oligo is synthesized so that it is present as a duplex when the reaction is complete. TOCE fluorescence signals are only detectable below the melting temperature of the catcher duplex, so that different probes on the same fluorescence channel can be distinguished by detection at different temperature levels or by melt analysis. Use of different "catcher" oligos with different melting temperatures of the respective duplexes allows different target sequences to be detected in a single sample at different temperatures.

Similar to the TOCE principle, a commercially availabe multiplex digital PCR assay (Stilla Technologies, France) uses a non-fluorescent target-specific oligonucleotide probe (called "crystal flex probe") with a tag. The Tag is split off from the probe and then serves as a primer to trigger the fluorescence of the universal reporter (EP 4372100 A1). Multiplexing is supported by using two different fluorescent dyes in the sense of a color combination coding. With a filter set that can distinguish up to 7 dyes, up to 21 plex reactions can be carried out.

Another commercially availabe multiplex-PCR called "TAGS" ("Temperature-Activated Generation of Signal"; Roche Diagnostics International AG, Switzerland; WO 2018/050828 A1) allows up to 15 targets to be detected simultaneously in a sample. In "TAGS", a Taqman probe probe is provided with a tag sequence in addition to the usual fluorophore/quencher labels, either at the 5' end, at the 3' end or internally, to which a complementary oligonucleotide (quencher tag) binds that is provided with an additional quencher. Below the melting temperature of the quencher tag, the fluorescence signal is therefore additionally suppressed by the quencher tag. Accordingly, once the reaction is finished, the fluorescence signal can be modulated by measuring at different temperatures or by melt analysis.

In still another method, a multi-component nucleic acid complex (MNA complex) is used in a so-called "PlexZyme" multiplex qPCR system (SpeeDx Pty. Ltd, Australia; see, e.g. WO 2007/041774 A1). In this system the primers amplify the target nucleic acid sequences and generate amplicons that serve as a template for the formation of a so-called "PlexZyme", nucleic acid enzymes which form from component partzymes only when target amplicons are present. As soon as the partzymes have assembled to form PlexZyme enzymes, universal probes bind and the enzymatic cleavage of the probes between fluorophore and quencher dye pairs generates fluorescence. The changes in fluorescence enable the detection and/or quantification of the target nucleic acid in real time. According to the manufacturer, the technology can distinguish up to two different parameters per channel. This technology is being used commercially for the first time as an in-vitro diagnostic (IVD) in the "RespiV Plusplex" assay.

It is an object of the invention to provide new means and methods for the improvement of ncleic acid detection. **In** particular, it is an object of the invention to increase the multiplexing potential of nucleic acid detection methods.

For solving the problem the invention provides, in a first aspect, a reporter probe for the detection of a target nucleic acid, the reporter probe comprising
i) a first oligonucleotide, the first oligonucleotide comprising, consecutively in 5'-3' direction, a) a first nucleotide sequence complementary to a first target region of the target nucleic acid and b) a second nucleotide sequence not complementary to the target nucleic acid, and
ii) a second oligonucleotide, the second oligonucleotide comprising, consecutively in 5'-3' direction, c) a third nucleotide sequence comprising a fluorophore-quencher pair, d) a fourth nucleotide sequence not complementary to the target nucleic acid but reverse complemantary to the second nucleotide sequence of the first oligonucleotide, and e) a fifth nucleotide sequence complementary to a second target region of the target nucleic acid, the second target region being adjacent to the first target region of the target nucleic acid.

The reporter probe of the invention comprises or is composed of a pair of single-stranded oligonucleotides configured to partially anneal to each other and to different but adjacent target regions of the same strand of a target nucleic acid, thus forming a triplex composed of the first and second oligonucleotide and the target nucleic acid. For this purpose, the first oligonucleotide ("head oligo") of the pair of oligonucleotides has a 5' segment ("targeting portion"; first nucleotide sequence) being configured to anneal to a first target region of a target nucleic acid, and a 3' segment ("overlap portion"; second nucleotide sequence) being configured not to anneal to the target nucleic acid but to part of the second oligonucleotide. The second oligonucleotide ("tail oligo") has a 3' segment ("targeting portion"; fifth nucleotide sequence) being configured to anneal to a second target region of the target nucleic acid adjacent to the first region, a 5' segment ("labeling portion" or "tagging portion"; third nucleotide sequence) not being complementary und thus not annealing to the target nucleic acid and comprising a fluorophore-quencher pair, and an intermediate segment ("overlap portion"; fourth nucleotide sequence) in between the 3' and the 5' segments that is reverse complementary to the 3' segment of the first oligonucleotide, such that, when both the first and second oligonucleotid are bound (annealed) to the respective adjacent target regions on the target nucleic acid, the oligonucleotides overlap, i.e. anneal to each other, with the "overlap portions". Head and tail oligonucleotide are configured in such a way that, when they anneal to each other with the overlap portions, the labeling portion forms a freely protruding single-stranded overhang. The melting temperature of the "overlap portions" is preferably chosen such that the first and second oligonucleotide only anneal with the overlap regions when bound to the target sequence. In such a bound state, the labeling portion of the tail oligo comprising the fluorophore-quencher pair (tag) extends from the overlap portion, forming a single-stranded branch sequence branching from the target nucleic acid. The tag is preferably configured to not fluoresce as long as the labeling portion is in its single-stranded configuration, but only when it is completed to a double-strand, e.g. by a suitable polymerase, to at least a sufficient extent to enlarge the distance between fluorophore and quencher to allow fluorescence of the fluorophore. The reporter probe of the invention significantly improves the specificity of nucleic acid detection, because the oligonucleotide pair only binds to the target molecule forming a triplex when both target regions are present on the target molecule in the required spatial relation to each other.

The reporter probe of the invention is useful for the detection and/or quantification of a nucleic acid or nucleic acids, in particular DNA, in a biological sample, for example, a body fluid sample, in vitro. The reporter probe is also particularly useful in a method for the detection and/or quantification of a nucleic acid in vitro, the method either including or not including amplification of the nucleic acid. The reporter probe of the invention has a very wide range of applications and can be used in a variety of methods involving tag-probe labeling of a nucleic acid, for example, in a polymerase chain reaction (PCR) method, for example qualitative or quantitative real-time PCR (rtPCR), or digital PCR, involving cycles of several (e.g. three) temperature steps. Alternatively, the reporter probe of the invention can be used in isothermal amplification methods, e.g., in Loop-mediated isothermal amplification (LAMP). RNA templates can be detected in reverse-transcriptase PCR (RT-qPCR). The reporter probe of the invention can be used in the context of singleplex or multiplex methods, for example PCR methods or isothermal detection methods. The reporter probe of the invention can be configured to allow the generation of temperature-dependent fluorescence signals. Further, the reporter probe can advantegeously be used in combination with a Taqman assay. The reporter probe of the invention does not require use of a polymerase having 5'-exonuclease activity, and can also be used with non-hydrolyzing polymerases (e.g., phi29, Bst, Bst 2.0, (Deep) Vent (-exo)). The reporter probe of the invention is universally applicable and can, inter alia, be used in connection with genetic studies in different areas, for example, in the field of infectiology or genetic polymorphisms (e.g. single-nucleotide polymorphisms, SNPs), or in plant or animal research.

The term "reporter probe" refers to an oligonucleotide, e.g., DNA oligonucleotide, that is configured to specifically bind (anneal) to a target sequence in a target nucleic acid, and that is provided with a detectable label, e.g. a fluorescent reporter dye (fluorophore) or a flurophore-quencher pair, with the help of which the target nucleic acid can directly or indirectly be detected and/or quantified. The term "reporter probe" as used herein encompasses a set of separate oligonucleotides, e.g. a pair of oligonucleotides, being configured to function together as a reporter probe. The separate oligonucleotides may be configured to include portions (sequence sections) allowing the separate oligonucleotides to specifically anneal at least partially to each other under specific circumstances. For ease of reference the term may also be used for a reporter probe of the invention being modified during use of the probe in a nucleic acid detection method, e.g. by extension of one of the oligonucleotides or by cleaving-off of the dual-labeled double-stranded part of the probe.

The term "oligonucleotide" (also abbreviated "oligo") refers to a comparatively short chain of nucleotides. An oligonucleotide is preferably composed of not more than 200 nucleotides, preferably not more than 150 or not more than 100 nucleotides. The term encompasses single- or double-stranded DNA or RNA or DNA-RNA-hybrids. Further, the term encompasses oligonucleotides being modified in the phosphate, sugar or nucleobase component, e.g. oligonucleotides including modified nucleotides or nucleotide analogs, for example, locked nucleic acid nucleotides (LNA nucleotides), phosphorothioate nucleotides (PS nucleotides), phosphorodithioate nucleotides (PS2 nucleotides), methylphosphonate nucleotides (MP nucleotides), 2'-O-methyl (2'-OMe), 2'-O-methoxyethyl (2'-MOE), or 2'-Fluoro (2'-F) nucleotides (see, e.g., Mohammed, A.A., AlShaer, D., Al Musaimi, O. ,Oligonucleotides: evolution and innovation. Med Chem Res 33, 2204-2220 (2024), doi:10.1007/s00044-024-03352-7). LNA nucleotides are RNA nucleotides in which the ribose ring is modified with a bridge (e.g. methylene bridge) connecting the 2'-O and the 4' carbon. LNA nucleotide containing oligonucleotides show higher thermal stability when hybridized to a complementary nucleic acid strand compared to a corresponding DNA oligo, for example, and thus have a higher melting temperature (Tm) compared to the DNA oligo. The term "oligonucleotide" thus encompasses the term "LNA oligonucleotide", the latter term referring to an oligonucleotide comprising or being composed of LNA nucleotides. Phosphorothioate-, Phosphorodithioate and methylphosphonate-modified oligonucleotides are oligonucleotides comprising nucleotides linked by phosphorothioate (PS), phosphorodithioate (PS2) or methylphosphonate (MP) bonds instead of phosphodiester bonds, i.e., in which a sulfur atom or a methyl group is substituted for a non-bridging oxygen (or two sulfur atoms are substituted for two non-bridging oxygens in case of phosphorodithioate groups) in the phosphate backbone. Presence of PS nucleotides, for example, lowers the Tm of oligonucleotides. In 2'-O-methyl (2'-OMe), 2'-O-methoxyethyl (2'-MOE), or 2'-Fluoro (2'-F) nucleotides, a methyl group, methoxyethyl group or fluorine atom is bound to the 2'-carbon atom of the sugar component. Nucleotides with combined modifications may also be included in an oligonucleotide, e.g., a phosphorothioate-modified nucleotide having a 2'-OMe group. The term "oligonucleotide" also encompasses oligos modified, at the 3' end, in the residue at the 3' carbon atom of the sugar component, in particular with a 3' terminator, e.g. an oligo including a 3' methyl-phosphorothioate (MPS) nucleotide, a 3' phosphate nucleotide, or a 3' C3 spacer nucleotide, i.e. a nucleotide being substituted, at the 3' position, with a three-carbon (C3) spacer.

The terms "target nucleic acid" or "target sequence" refer to a nucleic acid or nucleotide sequence of interest for detection and/or amplification.

The terms "bind", "anneal", "hybridize" in the context of an attachment of a reporter probe of the invention or one of its parts, sections or regions to a nucleotide sequence or to a section or region on a nucleic acid, e.g. to a target nucleic acid or to a part of an oligonucleotide forming part of the reporter probe of the invention are used interchangeable here and refer to the reversible specific attachment of a nucleic acid or part thereof to another nucleic acid or part thereof based on Watson-Crick base-pairing due the complementarity of the sequences, leading to the formation of a double-stranded nucleic acid from complementary single stranded nucleic acids. A first nucleic acid, nucleotide sequence, region or section of a nucleic acid may non-covalently bind to a second nucleic acid, nucleotide sequence, region or section of a second nucleic acid due to the complementarity of the nucleotide sequences of the two nucleic acids. The complementarity of nucleotides is a well-known to the skilled person, and is due to the formation of hydrogen bonds between opposing nucleobases in a double strand of DNA, RNA or DNA/RNA hybrid. It is known, that the nucleobase adenine (A) pairs with thymine (in DNA) or uracil (in RNA), and cytosine pairs with its complement guanine, for example. The term, according to which a first sequence is "complementary" to a second sequence such that the two sequences anneal to or hybridize with each other, is not to be construed as meaning that there has to be a 100% match of any of the nucleotides of the first sequence with a respective complementary nucleotide in the second sequence, but that there is enough complemantarity between the two sequences to lead to the specific annealing (hybridization) of the two sequences at a given temperature. Hybridization between two single-stranded nucleic acids may thus occur between two nucleic acid strands perfectly matched or substantially matched with some mismatches. Whether or not, or to which degree hybridization occurs may depend on hybridization conditions ("stringency") such as temperature, concentration of components (e.g. salt and detergent concentration), hybridization times, buffer components, pH and ionic strength, particularly on temperature. "High stringency", for example, refers to conditions in a solution under which two nucleic acids hybridize only where the nucleic acids share a high degree of complementarity (e.g. at least 50-99%) over a considerable sequence length. In order to hybridize relatively short stretches of complementary nucleic acids low stringency conditions are suitable. For hybridization conditions see, for example, Green M.R., Sambrook J, 2012, Molecular Cloning, A Laboratory Manual, Cold Spring Harbor Laboratory Press, 4th edition; Anderson M.LM., (1998), Nucleic Acid Hybridization, Garland Science.

The term "reporter probe targeting a target nucleic acid" means that the reporter probe is configured to target the nucleic acid, i.e. to specifically anneal to or hybridize with the target nucleic acid under suitable conditions. For being able to target the nucleic acid, the reporter probe of the invention comprises a first nucleotide sequence as a part of the first oligonucleotide and a fifth nucleotide sequence as a part of the second oligonucleotide, both the first and fifth nucleotide sequence being complementary to different regions of the target nucleic acid.

The term "complementary to a target region" means complementary to the nucleotide sequence of the target region. The term does not exclude that a number of or individual nucleotides of a given sequence are not complementary, i.e. do not follow Watson-Crick base pairing rules. "Complementary to a target region" in relation to a nucleotide sequence includes any complementarity being sufficient for the nucleotide sequence to anneal to (hybridize with) the target sequence.

The term "not complementary" or "non-complementary" refers to two nucleic acids or sequences that do not share nucleobases that can form Watson-Crick bonds or that share only a low amount of complementary nucleobases, e.g. less than 30 %, preferably less than 25%, 20% or less than 15%, more preferably less than 10% or 5% complementary nucleobases, i.e. nucleobases that can form a double-stranded nucleic acid by Watson-Crick base pairing.

Term like "a nucleotide sequence complementary" or "a nucleotid sequence not-complementary" refer to nucleotide sequences being configured, in terms of the order of nucleotides making up the nucleotide sequence, to be complementary or not complementary to a given (target) nucleotid sequence.

The terms "being configured to anneal" or "being configured to hybridize" in relation to a given nucleic acid or nucleotide sequence refers to the fact that a specific nucleotide sequence has been chosen or synthesized as to allow for the annealing of the nucleic acid or nucleotide sequence to (or hybridization with) another (complementary) nucleici acid or nucleotide sequence.

The term "melting temperature", Tm, in relation to a double-stranded nucleic acid, e.g. double-stranded DNA (dsDNA) refers to the temperature at which half of the nucleic acid strands are in a single-stranded or random coil state. The melting temperatures depends on the length of the double-stranded nucleic acid and the nucleotide sequence, e.g. its GC content. Further, the melting temperature can also depend from the presence and/or the kind of fluorophore. The term "melting" in relation to a double-stranded nucleic acid refers to the separation of the two strands of the double-stranded nucleic acid from each other. The term "denaturation" may also be used synonymously. A Tm of, for example, 65 °C given for a specific region of a reporter probe, e.g., a targeting region (targeting portion) being complementary to a target sequence or region of a target sequence, would thus mean that about 50 % of the probe form a duplex with the target sequence or region of the target sequence at a temperature of 65 °C.

The term "adjacent" in relation to the arrangement of a first nucleotide sequence or region of a nucleic acid in relation to a second nucleotide sequence or nucleic acid region means that the first and second nucleotide sequence or the first and second nucleic acid region are in close proximity to each other. This includes that the nucleotide sequences or regions directly adjoin or follow each other, but encompasses that the nucleotide sequences or regions are slightly spaced apart from each other, e.g., by one, two, three, four or up to ten nucleotides, preferably not more than 8, more preferably not more than 6 nucleotides, particularly preferred by one, two, or three nucleotides.

The term "consecutively in 5'-3' direction" in relation to the nucleotide sequences of the reporter probe does not exclude the presence of short spacer sequences of 1-5, preferably 1-4, more preferably 1-3 nucleotides length between the nucleotide sequences, e.g., between the targeting portions and overlap portions.

Terms like "configured such that" or "configured in a manner that" in relation to the reporter probe of the invention refer to the physical configuration of the probe, e.g., in terms of the nucleotide sequences (sequence length, nucleotide composition and order) making up the probe and/or the kind and arrangement of the fluorophore and quencher.

The term according to which "the tag is configured to not fluoresce as long as the labeling portion is in its single-stranded configuration, but only when it is completed to a double-strand" includes that the fluorescence of the fluorophor (dye) is quenched by the quencher as long as the labeling portion of the reporter probe is in a random coil configuration, for example, due to its single-stranded configuration and the close proximity of the fluorophore and the quencher in this configuration, and that fluorescence occurs as soon as the distance between fluorophor and quencher is increased by the formation of a double-strand so that the fluorescence of the fluorophor is no longer quenched by the quencher. In this context, it is to be noted that "an at least essentially double-stranded state of the third nucleotide sequence" refers to a configuration in which the third nucleotide sequence has an essentially double-stranded state, i.e., a state in which at least enough of its nucleotides are paired with complementary nucleotides to ensure a distance between fluorophore and quencher sufficient to substantially eliminate the quenching effect of the quencher.

The term "polymerase chain reaction", PCR, refers to a method for the amplification of a specific segment of DNA. PCR involves the use of short (e.g., 15-30 bases long) synthetic DNA fragments ("primers") to select a segment of DNA to be amplified, nucleotides (dNTPs) as building blocks, and a heat-stable polymerase (e.g. *Taq* DNA polymerase) for the polymerization of new DNA strands. PCR is based on three steps for DNA synthesis: (1) melting (denaturation) of the target (template) nucleic acid into single strands, (2) annealing of primers to each of the original strands and (3) extension of the newly synthesized DNA strands from the primers. The reaction is carried out in a suitable buffer solution, and in the presence of Magnesium ions. Most PCR methods use thermal cycling, i.e. repeated cycles of heating and cooling to different temperatures for optimal conditions of the reactions involved.

The terms "real-time PCR", rtPCR, or "quantitative PCR", qPCR, refer to a PCR method for the detection and quantification of nucleic acids, in which the amplification course of the target DNA can be monitored in real time. The kinetics of the rate of product synthesiz during the PCR reaction is used to quantitate the amount of template present. "Dye-based" real-time PCR is based on the incorporation of fluorescent dyes into double-stranded DNA and real-time measurement of the fluorescence, whereas "probe-based" real-time PCR uses fluorescence-labeled DNA probes. Probe-based qPCR allows multiplexing. Real-time PCR is not to be confused with "reverse transciption PCR", RT-PCR, which uses reverse transcription of RNA into DNA (so-called "complementary DNA" or cDNA) and subsequent PCR amplification of the DNA.

The term "digital PCR" (dPCR) refers to a quantitative PCR method in which the PCR solution is partitioned into a large number (e.g. 20000) of individual separate subsamples (e.g. small droplets) prior to amplification. Fluroescent probes are used to identify amplified target DNA, and positive and negative partions are identified by the presence or absence of fluorescence,. Quantification is performed using Poisson statistical data analysis.

The term "Taqman assay" refers to a quantitative PCR assay, in which a hydrolysable fluorogenic oligomer (Taqman probe) is used that is configured to hybridize within a target sequence defined by PCR primers and has a fluroescent reporter dye at the 5' end and a quencher at the 3' end. Replication of the target sequence during PCR by the polymerase results in the cleavage of the probe due to the 5'exonuclease activity of the polymerase. This cleavage separates the quencher and the dye whereby the reporter dye starts to emit fluorescence.

The terms "multiplex detection", "multiplex amplification", "multiplex assay" or "multiplex PCR" refer to the detection and/or amplification and/or quantification of more than one target nucleic acid in a single reaction mixture. Multiplex PCR uses multiple primer pairs. The term "multiplex" thus refers to the simultaneous evaluation (e.g. detection) of more than one, i.e. two or more, targets of interest (e.g. analytes or target nucleic acids or sequences). The term "singleplex", on the other hand, refers to the evaluation of only one target.

The term "isothermal amplification" (isothermal nucleic acid amplification techniques, (INAATs refers to methods of amplifying a nucleic acid, e.g., DNA, without thermal cycling, i.e., at a constant temperature. INAATs require the use of a polymerase with strand-displacement activity, e.g. Bst DNA Polymerase, Klenow exo⁻ and Phi29 (EquiPhi29). Examples of isothermal amplification techniques are Loop-mediated isothermal amplification (LAMP), Rolling circle amplification (RCA), Multiple displacement amplification (MDA), Recombinase polymerase amplification (RPA), Helicase-dependent amplification (HDA), Nucleic acid sequence-based amplification (NASBA), Transcription-mediated amplification (TMA), Strand displacement amplification (SDA), and Exponential amplification reaction (EXPAR).

The terms "melting curve analysis", "melting analysis" or "melt analysis" refer to the assessment of the dissociation (denaturation) characteristics of a double-stranded nucleic acid, e.g., DNA, during heating. A melting curve analysis is, for example, often performed after amplification of a nucleic acid to evalutate the specifity of the amplicon and/or the presence of unspecific products or primer dimers.

The term "amplicon" refers to a nucleic acid, e.g. RNA or DNA, or a part therof, that is the source and/or the product of an amplification or replication.

The term "detection" in relation to a target nucleic acid refers to the determination of the presence or absence of the target nucleic acid in a sample, e.g. a biological sample. The term includes qualitative and quantitative determination of the target nucleic acid, and does not exclude the amplification of the target nucleic acid in order to enable or facilitate detection.

The term "quantifying" means the determination of the quantity, e.g. the concentration, of an analyte, e.g. nucleic acid, in a sample.

The term "fluorophore" refers to a fluorescent chemical compound, i.e. a compound reemitting light upon light excitation. Examples include Texas Red (CAS 82354-19-6), Fluorescein (FAM; CAS 2321-07-5) or derivatives thereof (e.g. 5-FAM (5-Carboxyfluorescein) or 6-FAM (6-Carboxyfluorescein), JOE (4',5'-dichloro-2',7'-dimethoxy fluorescein), VIC (2'-chloro-7'-phenyl-1,4-dichloro-6-carboxyfluorescein), Cyanine 5.5 (Cy5.5), Atto647N, and Atto620 (ATTO-TEC GmbH, Germany). The term "FAM" used herein encompasses the terms 5-FAM and 6-FAM, and may refer to 5-FAM, 6-FAM or mixtures thereof.

The term "quencher" refers to a chemical compound suppressing or reducing the fluorescence of a fluorophore. The term "dark quencher" refers to a quencher having no native fluorescence that absorbs excitation energy from a fluorophore nonradiatively (without reeimitting light).

The term "biological sample" refers to any material originating from a living organism. Examples include, but are not limited to, blood, serum, plasma, urine, lymph, saliva, tissue or organ samples, or samples of parts derived therefrom, e.g., cell cultures, protein or nucleic acid samples.

The term "nucleoside triphosphate", dNTP, refers to deoxynucleoside triphosphates, i.e. triphosphates of nucleosides. For the synthesis of DNA, the nucleoside triphosphates dATP (deoxyadenosine triphosphate), dGTP (deoxyguanosine triphosphate), dCTP (deoxycytosine triphosphate) and dTTP (deoxythymidine triphosphate) are the substrates of the DNA polymerase.

The term "polymerase" refers to an enzyme catalyzing the synthesis of DNA or RNA, in particular DNA. Unless the context clearly teaches otherwise, the term "polymerase" refers to a DNA polymerase, i.e. a polymerase for the synthesis of DNA. The term "heat-stable polymerase" refers to a polymerase from thermophiles, e.g. thermophilic bacteria or archaea. Examples are polymerases from Thermus aquaticus (Taq polymerase), Thermus flavus (Tfl polymerase), Thermus thermophilus (Tth polymerase), Geobacillus stearothermophilus (Bst Klenow fragment) or Pyrococcus furiosus (Pfu polymerase).

The term "Carbapenemase" refers to bacterial enzymes, from the group of β-lactamases, that break down and render inactive carbapenem antibiotics, a class of antibiotics used as a last resort in case of failure of other antibiotics in severe infections, thus rendering bacteria, e.g., some strains of gram-negative enterobacteria (abbreviated "CRE" = carbapenem-resistant Enterobacteriaceae or "CPE" = carbapenemase-producing Enterobacteriaceae) carbapenem-resistant.

The term "BlaGES" refers to Guiana extended-spectrum (GES) β-lactamases, or the respective coding gene. The terms "blaKPC", "blaOXA48", "blaNDM", "blaVIM", "blaIMP", "blaOXA23", and "blaOXA40/24" refer to different β-lactamases or their respective genes.

The term "Target-mediated Branched Overlap Extension" (TBOE) is used herein as a designation of and reference to the principle of the invention, using an extension of overlapping and annealing sequence portions branching from a target nucleic acid to which a reporter probe of oligonucleotides of the invention is bound.

In the context of the reporter probe of the invention comprising or being composed of a pair of first and second oligonucleotides, the following terms will be used for ease of reference and better understanding:
The first oligonucleotide will be referred to as "head oligo" (or "head oligonucleotide"), the second oligonucleotide will be referred to as "tail oligo" (or "tail oligonucleotide"). The term "targeting portion" will be used for parts or sections of the head and tail oligo being configured to specifically bind to the target nucleic acid. In order to distinguish between the targeting portions, the terms "head oligo targeting portion" and "tail oligo targeting portion" may be used. The term "overlap portion" will be used for the portions of the head and tail oligo being configured to anneal to each other. The term "head oligo overlap portion" may be used for this portion of the head oligo, the term "tail oligo overlap portion" for this portion of the tail oligo. The terms "labeling portion" or "tagging portion" will be used for the 5' segment of the tail oligo comprising the fluorophore-quencher pair. Labeling portion and overlap portion may together be termed "branch" or "branching portion". The term "part" may synonymously be used for the term "portion".

The invention provides a novel reporter probe that can advantageously be used in different methods for the detection and/or quantification and/or amplification of nucleic acids, for example, DNA or RNA molecules present in a biological sample. The reporter probe can also be used in multiplex nucleic acid detection or amplification methods, e.g. PCR methods. The sequences of the targeting portions of the oligonucleotide pair forming or being part of the reporter probe of the invention targeting, i.e. the nucleotide sequences being configured to anneal to the target nucleic acid, can be adapted to different target nucleic acids in order to enable detection of different target nucleic acids in the same sample. The binding affinities and thus the melting temperatures of the targeting portions of the oligonucleotides can be adjusted by the length and sequence of the targeting portions, and by nucleotide modifications, e.g. the incorporation of LNA or PS nucleotides. The reporter probes can, for example, be provided with different fluorophores in order to distinguish the probes and thus the different target DNAs from each other. Further, the reporter probes can be configured to have different melting temperatures, Tm, in relation to the duplex of head and tail oligo. The reporter probe of the invention can be combined with Taqman probes for single channel multiplexing at different temperatures. In a preferred embodiment of the invention, the probe only fluoresces when the labeling (branching) part of the tail oligo comprising the fluorophore-quencher pair is in a double-stranded state, e.g., as a result of the action of a polymerase present or added to the reaction mix that extends the 3'-end of the head oligo bound to the tail oligo with the branching sequence of the tail oligo as a template, the formation of fluorescence can be made dependent on the melting temperature of the duplex of the head and tail oligo including the double-stranded labeling part of the tail oligo. The melting temperature can, for example, be adjusted by the length and sequence of and/or the presence or absence of modified nucleotides, e.g. LNA or PS nucleotides, within the labeling portion and/or the overlap portion.

**In** a preferred embodiment of the reporter probe according to the invention the fluorophore-quencher pair is arranged in the third nucleotide sequence (the "labeling portion" or "tagging portion") of the second oligonucleotide (the "tail oligo) in such a manner that the fluorescence of the fluorophore is quenched in a single-stranded state, but not in an at least essentially double-stranded state of the third nucleotide sequence (the "labeling portion"). This can, for example, be accomplished by configuring the spatial distance between the fluorophore and the quencher, and the sequence of the tagging portion in a manner that the fluorophore and the quencher are in close proximity to each other in the single-stranded state, such that fluorescence of the fluorphore is quenched via contact and/or FRET ("Förster resonance energy transfer") quenching, whereas the fluorescence of the fluorophore is not quenched in the double-stranded state of the labeling portion, in which the fluorophore and quencher are spaced apart beyond the quenching distance. The quenching distance is dependend on the Förster radius of the respective compounds, i.e. the fluorophore and the quencher, and can, for example, be 18-22 nucleotides. As an example, the single-stranded form of the labeling portion may have a random-coil configuration, or may form a stem-loop (hairpin) structure, bringing the fluorophore and quencher into sufficient proximity. The formation of a double-strand with the aid of a polymerase may then lead to separation of the fluorophore and the quencher leading to fluorescence of the fluorophore. It is, of course also possible to provide a dual-labeled probe where the quencher or the fluorophore is cleaved off and released from the probe, e.g. by using a polymerase having 5' exonuclease activity.

The quencher-fluorophore pair used for dual-labeling of the reporter probe of the invention can be arranged in any suitable manner. In a preferred embodiment of the reporter probe of the invention, the quencher, is arranged at or near the 5' end of the third nucleotide sequence ("tagging portion") of the second oligonucleotide ("tail oligonucleotide"). In this embodiment, the fluorophore is preferably arranged at or near the 3' end of the the third nucleotide sequence.

In this context, the term "end" is not to be construed as meaning an open end, unless the context clearly teaches otherwise, but as a reference to the end of the respective portion or nucleotide sequence (e.g. the tagging portion) within the oligonucleotide. Alternatively, however, the fluorophore can be arranged at or near the 5' end of the third nucleotide sequence of the second oligonucleotide, while the quencher is arranged at or near the 3' end of the third nucleotide sequence of the second oligonucleotide.

The fluorophore and the quencher can be chosen appropriately depending on the intended use. The skilled person is familiar with suitable fluorophores and quenchers and also with suitable fluorophore and quencher pairs (see, e.g, Didenko, V. V., 2001, DNA Probes Using Fluorescence Resonance Energy Transfer (FRET): Designs and Applications, BioTechniques, 31(5), 1106-1121, doi: 10.2144/01315rv02). Suitable fluorophores are, for example, Texas Red (CAS 82354-19-6), Fluorescein (FAM; CAS 2321-07-5, 5-Carboxyfluorescein (5-FAM), 6-Carboxyfluorescein (5-FAM); the term "FAM" encompassing FAM, 5-FAM, 6-FAM or mixtures thereof, e.g. mixtures of 5-FAM and 6-FAM), JOE (4',5'-dichloro-2',7'-dimethoxy fluorescein), VIC (2'-chloro-7'-phenyl-1,4-dichloro-6-carboxyfluorescein), Cyanine 5.5 (Cy5.5), Atto647N, and Atto620 (ATTO-TEC GmbH, Germany). The fluorophore can be a fluorescent dye with large (long) Stokes shift (LS dyes), e.g., a Stokes shift of 80-145 nm in excitation/emission wavelengths, for example Atto490LS (ATTO-TEC-GmbH, Germany). The quencher is preferably a dark quencher. Suitable examples of dark quenchers are quenchers called "black hole quenchers" (Biosearch Technologies; see, e.g., US 7019129 B1) like BHQ-1 (CAS: 374591-92-1), BHQ-2 (CAS: 1214891-99-2) or BHQ-3. The dark quencher BHQ-2 may, for example, be combined with one of the above-mentioned fluorophores.

The first and second oligonucleotide of the reporter probe of the invention can be composed of DNA or RNA nucleotides, or a mixture of DNA and RNA nucleotides, or can comprise or being composed of modified nucleotides, e.g. LNA nucleotides, PS nucleotides, MP nucleotides, 2'-OMe nucleotides, 2'-MOE nucleotides, 2'-F nucleotides. The properties of the oligonucleotides, e.g., in terms of melting temperature between the oligos as well as between the reporter probe and the target sequences, can be adjusted not only by the sequence lengths and compositions but also by the composition of the nucleotides, e.g. the incorporation of modified nucleotides.

In a preferred embodiment of the reporter probe according to the invention, the first oligonucleotide (head oligo) is shorter in length than the second oligonucleotide (tail oligo), such that, when the head and tail oligo are annealed to each other with their overlap portions, the tail oligo protrudes single-strandedly with its labeling portion, or at least a part thereof, beyond the overlap portions connecting the two oligos.

In preferred embodiments the reporter probe of the invention is configured in such a manner that a first melting temperature, Tm1, of a duplex formed between the second and the fourth nucleotide sequences (the "overlap portions") of the first and second oligonucleotides (head and tail oligo) of the reporter probe is lower than a second and third melting temperature, Tm2, Tm3, of a duplex formed between either the first or the fifth nucleotide sequence (the "targeting portions") of the first and second oligonucleotides (head and tail oligos) of the reporter probe with the target nucleic acid. The melting temperature Tm1 between the overlap portions is set low enough to ensure that first and second oligonucleotide normally do not anneal to each other with the overlap portions without being bound to their respective target sequences. In preferred embodiments, Tm1 is in the range of 10-20 °C, preferably 12-18 °C, more preferably 14-17 °C, and Tm2 and Tm3 are each in the range of 60-80 °C, preferably 60-75 °C, 62-73 °C, 64-72 °C, 65-70 °C or 65-69 °C. Tm2 and Tm3 may be the same or different, but preferably differ by no more than 20 °C, more preferably no more than 15 °C, 10 °C or 5 °C. Preferably, Tm3, i.e., the melting temperature between the duplex formed between the target portion of the second oligonucleotide (tail oligo) and the target sequence, is higher, e.g. about 2-5 °C, preferably 3-4 °C, than Tm2, i.e., the melting temperature between the duplex formed between the target portion of the first oligonucleotide (head oligo) and the target sequence. This allows the tail oligo to bind first to the target sequence, before the head oligo. Subsequent binding of the head oligo to the target sequence is also promoted by binding to the overlap portion of the tail oligo. Tm2, the melting temperature of the duplex formed between the first nucleotide sequence (head oligo targeting portion) and the first target sequence on the target nucleic acid, may, for example, be 65-67 °C, e.g. 65 °C, Tm3, the melting temperature of the duplex formed between the fifth nucleotide sequence (tail oligo targeting portion) and the second target sequence on the target nucleic acid, 67-69 °C, e.g., 68 °C.

As already mentioned above, in preferred embodiments of the reporter probe of the invention, the reporter probe is configured in such a manner that a fourth melting temperature, Tm4, for a duplex formed between the head and tail oligo after extension of the 3'-end of the head oligo using the labeling portion of the tail oligo as a template and formation of a double-stranded labeling portion of the tail oligo, e.g. by a suitable polymerase, for example *Taq* polymerase, is preferably equal to or higher than Tm2 and Tm3. This is, however, not necessary the case, because, in some embodiments of methods using the reporter probe of the invention, for example in embodiments using *Taq* polymerase, the targeting portions are cleaved off from the reporter probe after initial binding to the target sequences releasing the double-stranded labeling portion and the overlap portions. Tm4 can be adjusted in a way that allows discrimination between different reporter probes of the invention and thus targets at different temperatures. For example, a set of three reporter probes for three different targets may have a Tm4 of 64-65°C for the first probe, 71-72°C for the second probe and 77-78°C for the third probe. In such a case, the difference between the different Tm4 values of the different reporter probes is preferably 6-7 °C.

In preferred embodiments, the fourth melting termperature, Tm4, is in the range of 60-85 °C, preferably 60-80 °C, 60-75 °C, 62-73 °C, 64-72 °C, 65-70 °C or 65-69 °C. As mentioned above, in case of a multiplex method, e.g. multiplex PCR, using different reporter probes of the invention, the Tm4 values of the different reporter probes used simultaneously in the multiplex method are adusted to be different from each other in a suitable manner to allow discrimination between the different reporter probes and thus targets. The reporter probe of the invention may be used in such a manner that the double-stranded branching portion, including the overlap portions of both the tail and head oligo, the labeling portion and the synthesized strand complementary thereto, is cleaved off from the rest of the reporter probe, i.e. the targeting portions.

The overlapping sequences preferably comprise 8-9 nucleotides, and the targeting portions of the head and tail oligo preferably comprise 20-30 nucleotides.

In a second aspect the invention relates to a method for the detection of a target nucleic acid, the method comprising the steps of
a) contacting the target nucleic acid with a reporter probe of the first aspect of the invention, described above, targeting the target nucleic acid, in the presence of a polymerase and nucleoside triphosphates, dNTPs, under conditions to allow nucleic acid synthesis by the polymerase, and
b) detecting the fluorescence of the reporter probe.

**In** the method of the invention, a target nucleic acid is, in a first step, contacted with a reporter probe of the invention, the reporter probe being configured to target the target nucleic acid, and comprising
i) a first oligonucleotide, the first oligonucleotide comprising, consecutively in 5'-3' direction, a) a first nucleotide sequence complementary to a first nucleotide sequence of a first target region of the target nucleic acid and b) a second nucleotide sequence not complementary to the target nucleic acid, and
ii) a second oligonucleotide, the second oligonucleotide comprising, consecutively in 5'-3' direction, c) a third nucleotide sequence comprising a fluorophore-quencher pair, d) a fourth nucleotide sequence not complementary to the target nucleic acid but reverse complemantary to the second nucleotide sequence of the first oligonucleotide, and e) a fifth nucleotide sequence complementary to a second nucleotide sequence of a second target region of the target nucleic acid, the second target region being adjacent to the first target region of the target nucleic acid.

The reporter probe is contacted with the target nucleic acid in the presence of a polymerase, preferably a heat-stable polymerase, and nucleoside triphosphates, dNTPs, under conditions to allow nucleic acid synthesis by the polymerase.

In the presence of the target nucleic acid, both the first and second oligonucleotide anneal, with their respective targeting portions, which are complementary to the target nucleic acid, to the first and second target region on the target nucleic acid. First and second oligonucleotide are preferably configured such that the second oligonucleotide ("tail oligo") comprising the fluorophore-quencher pair anneals to its cognate target sequence at a higher temperature than the first oligonucleotide ("head oligo"). Both the first and second oligonucleotide comprise regions that are non-complementary to the target nucleic acid. The 3'-end ("head oligo overlap portion") of the first oligonucleotide (head oligo) is non-complementary to the target nucleic acid but revers complementary to the fourth nucleotide sequence ("tail oligo overlap portion") of the second oligonucleotide ("tail oligo"), to which it anneals when both oligonucleotides are bound to the target nucleic acid. In this state, the oligonucleotide pair forms a triplex with the target nucleic acid, annealed to the target nucleic acid with the targeting portions, and branching from the target nucleic acid with the overlap portions and the third nucleotide sequence of the second oligonucleotide ("labeling portion"). While the overlap portions anneal to each other forming a double-stranded branch part, the third nucleotide ("labeling portion") is single-stranded.

In the method of the invention, the fluorescence of the reporter probe is detected.

Since the reporter probe of the invention used in the method of the invention is configured such that fluorescence light is only or at least predominantly emitted when the fluorophore and quencher of the fluorophore-quencher pair included in the third nucleic acid (the "labeling portion") are distanced away from each other by the formation of a double-strand from the single-stranded labeling portion due to the activity of a suitable polymerase, and the formation of such a double-stranded labeling portion depends on the binding of the reporter probe to the target nucleic acid, fluorescence will only be detected when the reporter probe did bind to the target nucleic acid. The polymerase used in the method of the invention may or may not have exonuclease activity, e.g. 5' exonuclease activity. If the polymerase has exonuclease activity, the part of the reporter probe branching from the target nucleic acid and comprising the fluorophore-quencher pair may be released from the target nucleic acid.

In a preferred embodiment of the invention the polymerase serves to extend the 3' end of the first oligonucleotide with the third nucleotide sequence comprising the fluorophore-quencher pair as a template and synthesizes the complementary strand to the third nucleotide sequence, such that the portion of the probe comprising the fluorophore-quencher pair becomes double-stranded, leading to a sufficient spatial separation of the fluorophore-quencher pair and to fluorescence of the fluorophore. In this embodiment, a target nucleic acid can, for example, be detected in a biological sample without a prior amplification step.

In a preferred embodiment of the method of the invention the method further comprises the step of amplifying the target nucleic acid before or during step b) of detecting the fluorescence of the reporter probe of the invention. In this embodiment of the method of the invention, the target nucleic acid is amplified prior to or during detection of the fluorescence generated by the reporter probe of the invention. The amplification can be carried out under thermocycling or isothermal conditions.

In a particularly preferred embodiment of the method of the invention the method comprises an amplification step under thermocycling conditions, and is preferably a polymerase chain reaction (PCR) method. In this embodiment, the target nucleic acid is, in step a), contacted with at least one primer pair, preferably a primer pair comprising a forward and reverse primer. The primers of the primer pair are configured to specifically bind to flanking regions of the target nucleic acid.

Further, in this embodiment of the method of the invention preferably comprises at least one cycle of the steps of (a) denaturation, (b) reporter probe annealing, (c) primer annealing, and (d) extension, preferably a plurality of cycles, e.g. 20-50 cycles, preferably 30-50 cycles, of the steps of (a) denaturation, (b) reporter probe annealing (c) primer annealing, and (d) primer extension according to standard PCR procedures, wherein, in the denaturation step (a) a double-stranded DNA target nucleic acid is heated to separate the strands of the target nucleic acid, in the annealing step (b) the head and tail oligos bind to the target regions on the target nucleic acid, in the annealing step (c) primers, i.e. short DNA molecules, bind to flanking regions of the target DNA, and in the extension step (d) DNA polymerase extends the 3' end of each primer along the template strand. The steps (a), (b), (c) and (d) are performed at different temperatures, e.g. step (a) at 94-98 °C, step (b) at 65-69 °C, step (c) at 45-60 °C (about 2-5 °C below the lower Tm of the primers), step (d) at 70-75 °C. Step (d) is carried out in the presence of free nucleoside triphosphate molecules (dNTPs) and a heat-stable polymerase, e.g. *Taq* polymerase. All steps are carried out under suitable reaction conditions (e.g. in terms of the buffer solution, MgCl₂ concentration etc).

A further preferred embodiment of the method of the invention comprises the following steps:
(a) 5 s denaturation at 95 °C;
(b) 5 s reporter probe annealing at 68 °C;
(c) 30 s primer annealing at 60 °C; and
(d) 5-10 s extension at 75 °C.

In a further preferred embodiment the method of the invention is configured as a real-time PCR method or digital PCR method.

In a further preferred embodiment of the method of the invention, the amplification is carried out under isothermal conditions. In this embodiment the method can, for example, be configured as a Loop-Mediated Isothermal Amplification (LAMP) method. The skilled person is familiar with this method, which involves the use of 4-6 primers and a strand-displacing DNA polymerase. The reporter probe of the invention can advantageously be used as a reporter probe in this method to detect a target nucleic acid to be amplified, either at the end of the amplification process or during amplification.

The method of the invention can also be configured as any other isothermal amplification method (INAAT), for example, Rolling circle amplification (RCA), Multiple displacement amplification (MDA), Recombinase polymerase amplification (RPA), Helicase-dependent amplification (HDA), Nucleic acid sequence-based amplification (NASBA), Transcription-mediated amplification (TMA), Strand displacement amplification (SDA), and Exponential amplification reaction (EXPAR). The reporter probe of the invention can also be used as a reporter probe in this method.

In a particularly preferred embodiment of the method of the invention, the method is configured as a multiplex method using a plurality of reporter probes of the invention, for example 2-12, preferably 2-10 or 2-8, reporter probes of the invention, the reporter probes of the invention differing in the melting temperature Tm4 and/or in fluorophore color (i.e. the wavelength of the fluorescence emitted). Preferably, the multiplex method is a multiplex PCR, e.g. rtPCR or dPCR method, or a multiplex LAMP method.

In a further preferred embodiment of the method of the invention, the method is configured as a multiplex method, for example a multiplex PCR or multiplex LAMP method, using a reporter probe of the invention in combination with Taqman probes.

In a further aspect, the invention relates to a kit for the detection of a target nuclei acid, the kit comprising a reporter probe of the invention.

The kit of the invention preferably comprises components necessary for the method to be carried out, e.g., for any of the isothermal amplification methods mentioned above, or a PCR method, e.g., a real-time PCR (qPCR) method.

In a preferred embodiment of the kit of the invention the kit further comprises a polymerase, preferably a heat-stable polymerase, and nucleoside triphosphates, dNTPs.

In a preferred embodiment of the kit of the invention, the kit comprises a plurality of reporter probes of the invention, the reporter probes of the invention differing in the melting temperature Tm4 and/or in fluorophore color. The kit may, for example, comprise 2-12, preferably 2-10 or 2-8, different reporter probes of the invention.

In a further preferred embodiment the kit further comprises at least one primer, preferably at least one pair of primers configured to bind to the target nucleic acid. Depending on the number and configuration of the primers included in the kit, this embodiment is intended for an INAAT method (see above) or a PCR (e.g. qPCR) method.

In a still further aspect the invention relates to the use of a reporter probe of the invention for the detection of a target nucleic acid in a biological sample. Preferabyl, the detection of the target nucleic acid includes a step of amplifying the target nucleic acid.

In the following, the invention will be further described by way of example with reference to the accompanying figures and examples.

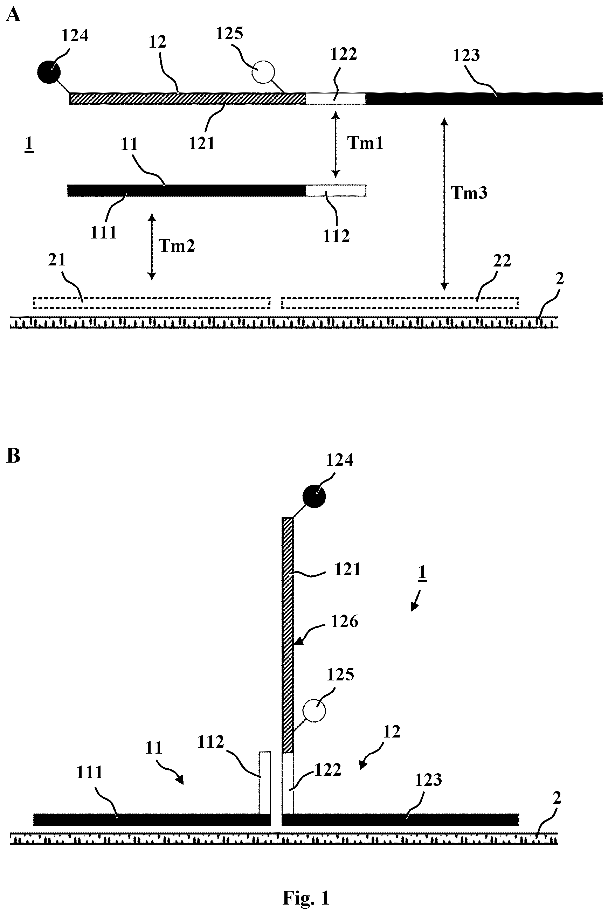

Figure 1. Simplified schematic representation of an embodiment of a reporter probe of the invention. A. Simplified scheme of the components of an embodiment of a reporter probe of the invention and binding properties of portions thereof in terms of melting temperatures. B. Simplified scheme of an embodiment of a reporter probe of the invention bound to a target nucleic acid.

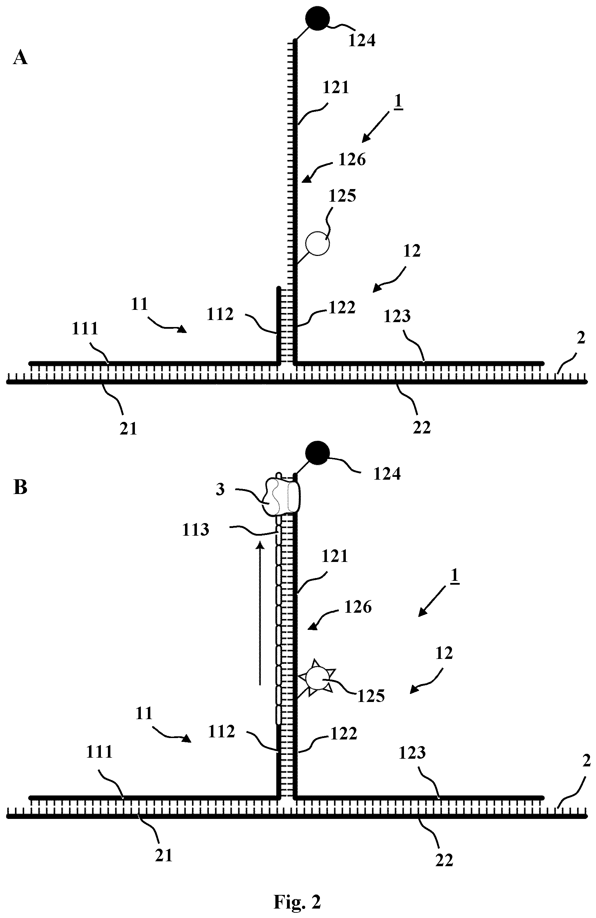

Figure 2. Schematic representation of the embodiment of a reporter probe of the invention depicted in Fig. 1 with crossbars symbolizing nucleobases and a line symbolizing the sugar-phosphate backbone. A. Reporter probe bound to a template strand of a target nucleic acid. B. Reporter probe with the branching double-stranded

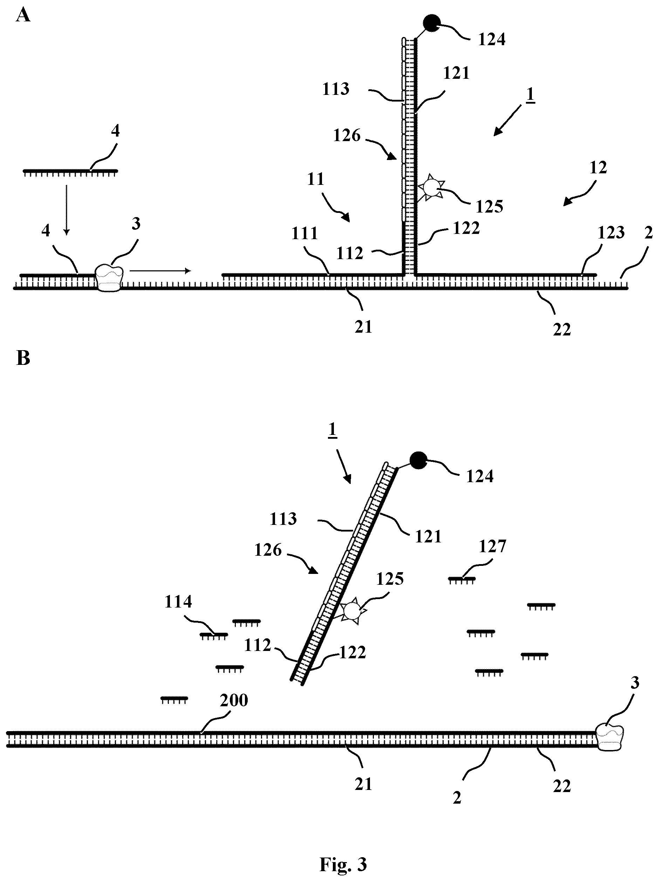

Figure 3. Schematic representation of the use of an embodiment of a reporter probe 1 of the invention in the context of a PCR method. A. Annealing and extension of primer. B. Degradation of reporter probe by cleaving-off of the double-stranded branch portion.

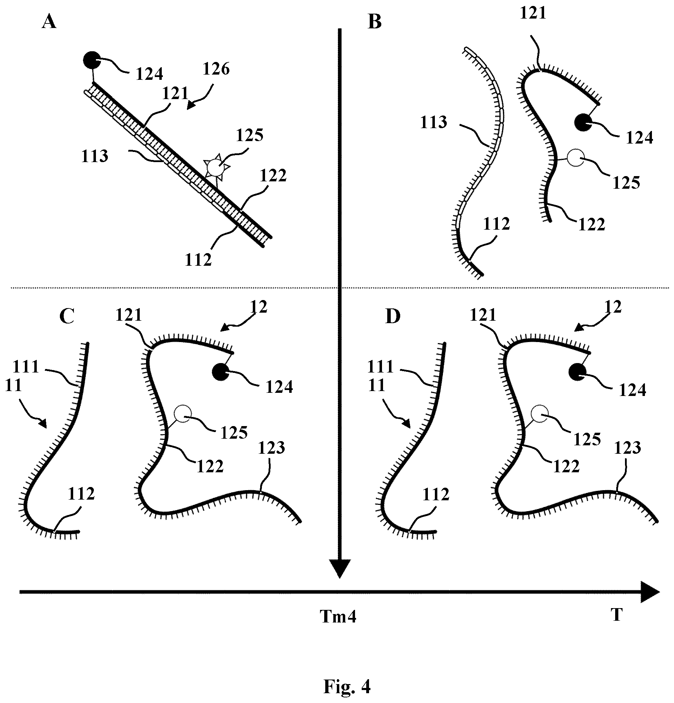

Figure 4. Schematic drawings of four possible states of the reporter probe as the result of an assay in which a polymerase having exonuclease activity has been use. A. Reacted probe at a temperature below Tm4; B. Reacted probe at a temperature above Tm4; C. Unreacted probe at a temperature below Tm4; Unreacted probe at a temperature above Tm4. A vertical arrow symbolizes the melting temperature Tm4. T = Temperature.

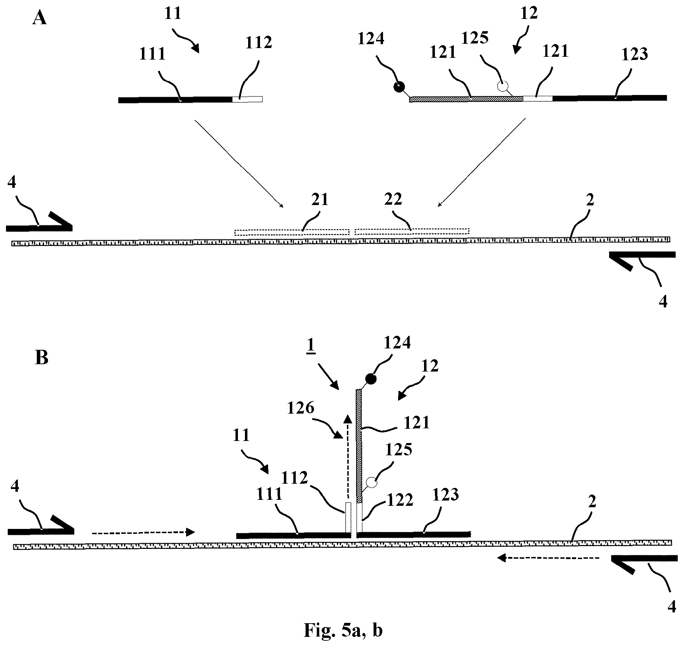

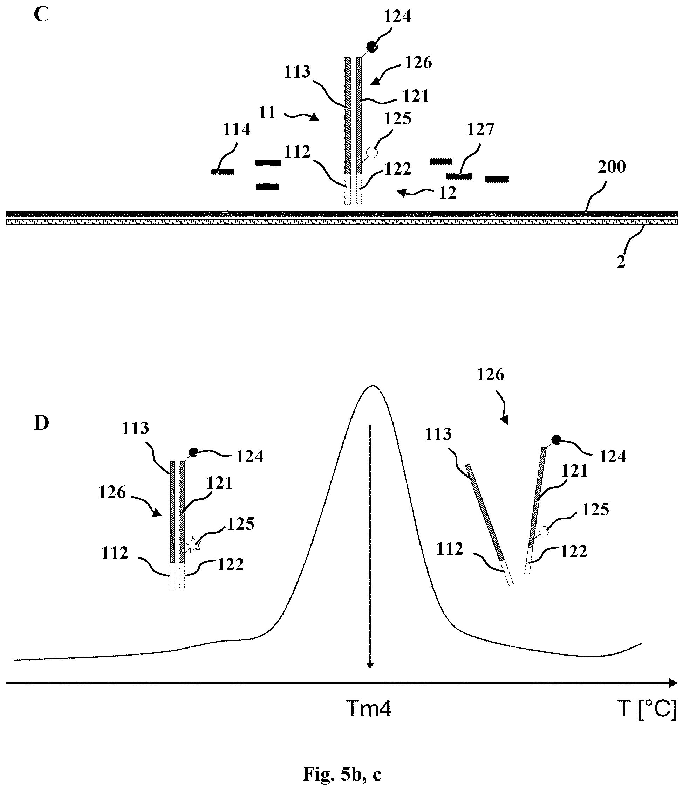

Figure 5. Schematic depiction of a PCR method using the reporter probe of the invention and a subsequent melting curve analysis using an embodiment of a reporter probe of the invention. A. Schematic representation of the annealing of primers and a reporter probe of the invention to a target nucleic acid. B. Primer elongation and elongation of the 3'-end of the head oligo of the reporter probe of the invention. C. Result of amplification. D. melting curve analysis.

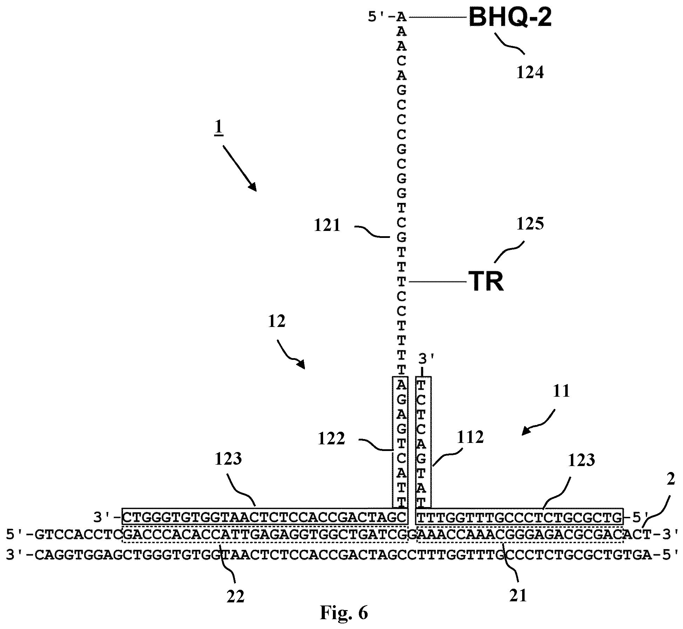

Figure 6 schematically shows an example of an embodiment of a reporter probe of the invention bound to a part of a BlaGES-5 gene. BHQ-2: Black Hole Quencher 2; TR: Texas Red.

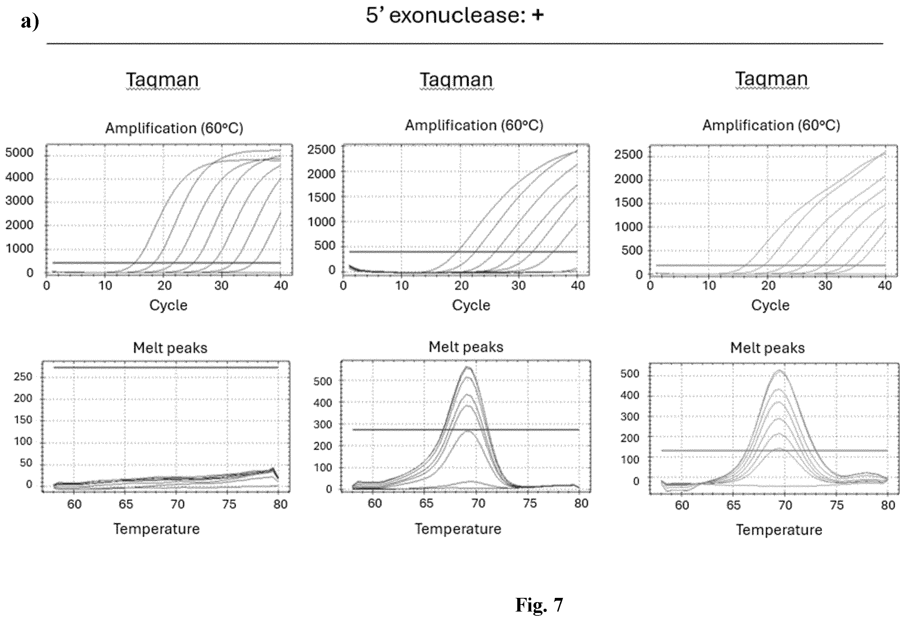

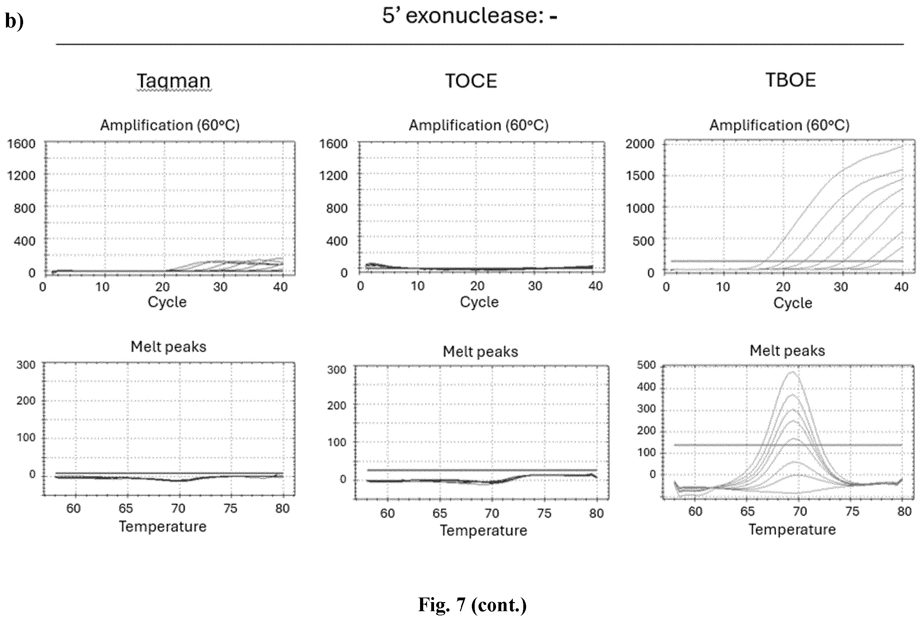

Figure 7. a) Comparison of a TaqMan (left column), TOCE (middle column) and TBOE (right column) based qPCR assay for detection of blaGES on a 10-fold dilution series. First row depicts amplification curves, y-axis: relative fluorescence units; x-axis: cycle. The second row depicts melt peaks. y-axis: negative delta of relative fluorescence units; x-axis: temperature. b) Same setup but using a polymerase without 5' exonuclease detection (Quantabio repliQa HiFi mastermix).

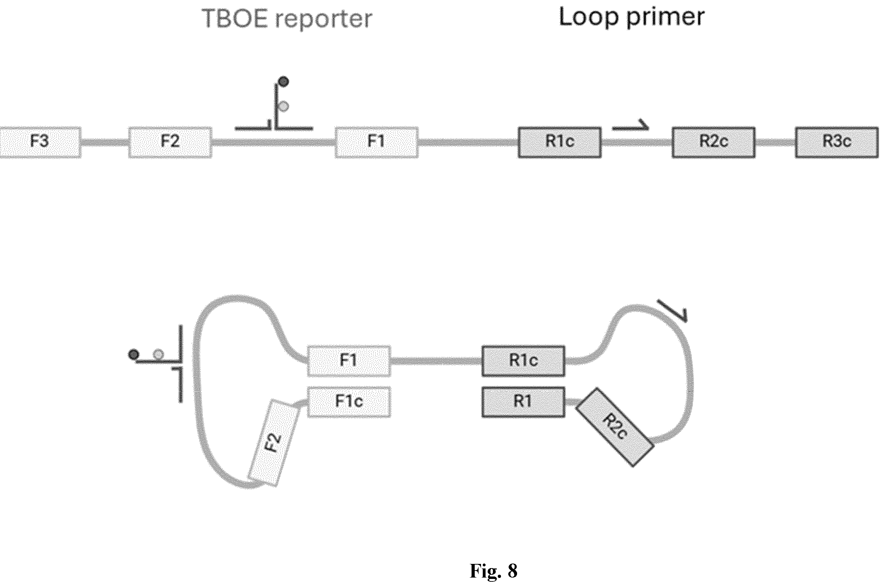

Figure 8. A classic LAMP design including a single loop primer was created to target blaGES, whereby one of the loop regions was made 60-80 bases long to incorporate the TBOE reporter.

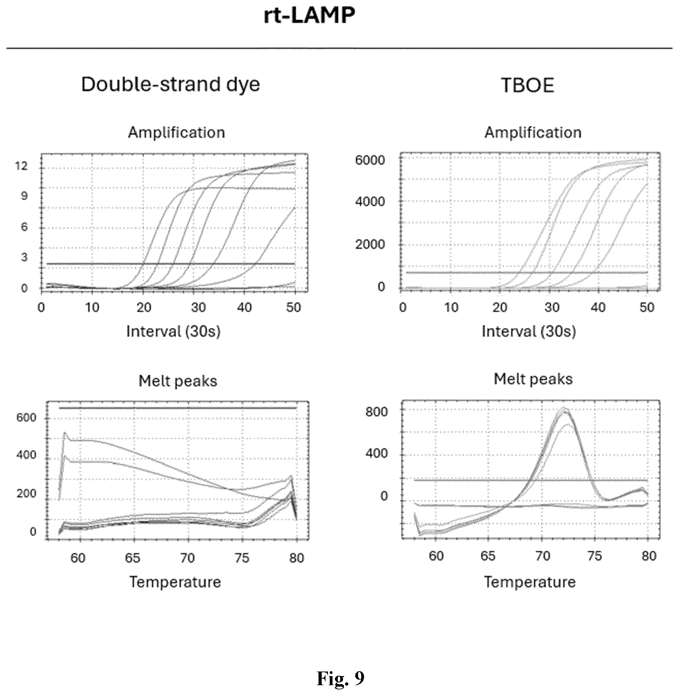

Figure 9. Amplification curves (first row) and melt peaks (second row) of a 10 fold dilution series of a blaGES positive Isolate. Graph setup is similar to figure 7. Detection of amplification, 30 second intervals were used between measurements.

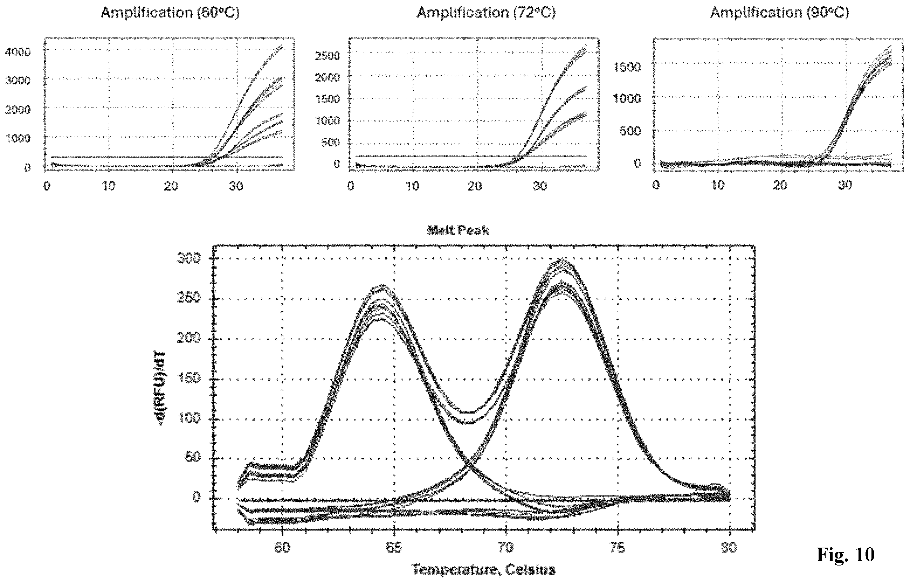

Figure 10. Amplification curves and melt peaks of the multiplex test (graph setup similar to figure 7). Each of the two TBOE based targets are allocated one individual Tm. Melt peak analysis allows differentiation of individual targets. Wells positive for both targets show a double-peak. The TaqMan based assay is not visible in melt-curve analysis.

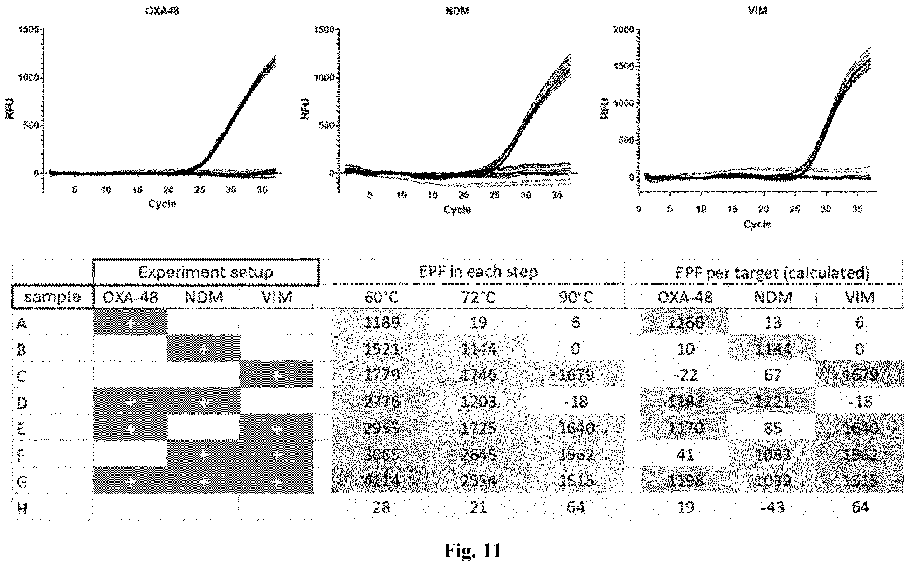

Figure 11. Signal deconfliction through detection at different temperatures during amplification. Individual target signals are extracted through subtraction (including correction factor) of raw RFU readings of individual temperature steps. First row shows individual amplification curves, reconstructed by inputting calculated RFUs into a new graph (Graphpad prism). The table shows experiment setup compared to end-point-fluorescence (EPF) of each raw temperature step and calculated EPF of signals after deconfliction. The calculated EPF values match the experimental setup of each sample.

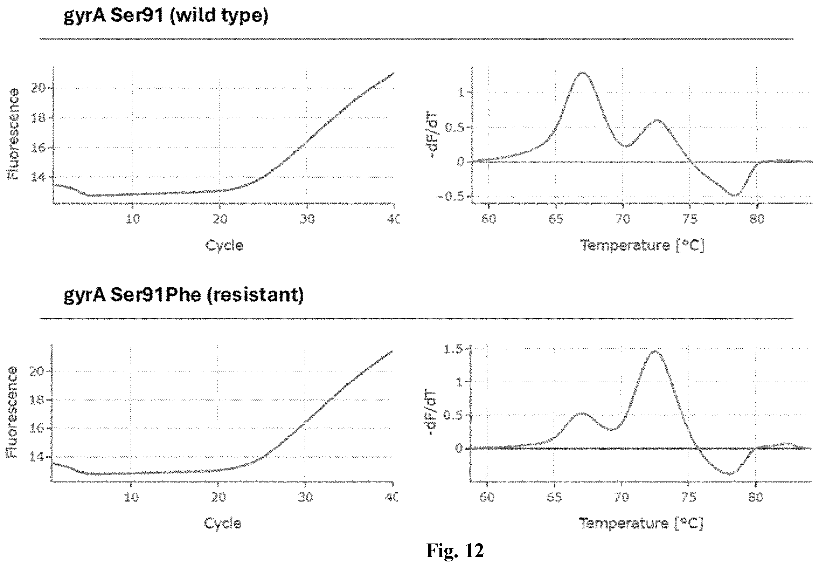

Figure 12. Exemplary amplification signals of the gyrA Ser91Phe single nucleotide polymorphism assay. The target sequence differs by only a single base, which is covered by two competing TBOE tails. The top row shows an exemplary N. gonorrhoeae isolate, negative for the mutation, with a dominant first melt-peak at 66.5°C. The second row shows an exemplary N. gonorrhoeae, positive for the mutation, showing a dominant second melt-peak at 72°C. Both samples show identical amplification curves.

Figure 1 shows a simplified schematic representation of an embodiment of a reporter probe 1 of the invention. Figure 1A shows a simplified scheme of the components of an embodiment of a reporter probe 1 of the invention and binding properties of portions thereof in terms of melting temperatures (Tm). Figure 1B shows a simplified scheme of an embodiment of a reporter probe 1 of the invention bound to a target nucleic acid 2. The reporter probe 1 of the invention depicted in Figure 1 is composed of a first oligonucleotide 11 ("head oligo" or "head oligonucleotide" in the following) and a second oligonucleotide 12 ("tail oligo" or "tail oligonucleotide" in the following. Head oligo 11 and tail oligo 12 are not covalently bound to each other. In the embodiment of the reporter probe 1 of the invention shown in Figure 1, the reporter probe 1 of the invention comprises five nucleotide sequences, two nucleotide sequences make up the head oligo, and three nucleotide sequences. The head oligonucleotide 11 comprises, consecutively in 5'-3' direction, a first nucleotide sequence 111 ("head oligo targeting portion") being configured, in terms of its nucleotide sequence, to specifically anneal to a first target region 21 on a target nucleic acid 2, and a second nucleotide sequence 112 ("head oligo overlap portion") being configured, in terms of its nucleotide sequence, to be reverse complementary to a corresponding overlap portion ("tail oligo overlap portion") 122 of the tail oligo 11. The tail oligonucleotide 12 comprises, consecutively in 5'-3' direction, a third nucleotide sequence 121, fourth nucleotide sequence 122 and a fifth nucleotide sequence. The third nucleotide sequence 121 ("labeling or tagging portion") carries a dual label, and comprises a fluorophore 125 and a quencher 124. In this embodiment, the quencher 124 is arranged at the 5' end of the third nucleotide sequence 121, and the fluorophore is arranged in a suitable distance from the quencher 124 near the 3' end of the third nucleotide sequence 121. The fourth nucleotide sequence 122 ("tail oligo overlap portion") is reverse complementary to the head oligo overlap portion. The fifth nucleotide sequence 123 is configured, in terms of its nucleotide sequence, to specifically anneal to a second target region 22 on a target nucleic acid 2. The first and second target region 21, 22 are adjacent to each other, i.e., either follow one another directly on the target DNA, or are separated by only a few nucleotides (e.g. 1-10 nucleotides, preferably not more than 8, more preferably not more than 6 nucleotides) from each other. The overlap portions 112, 122 are configured to be able to anneal to each other when the head and tail oligonucleotide 11, 12 are bound to their respective target regions 21, 22. In Figure 1A, arrows denote the parts of the reporter probe and the target nucleic acid that are configured to interact with each other due to the complementarity of the nucleotide sequences. Melting temperatures Tm1-Tm3 are mentioned as a parameter for these interactions. Tm1 denotes the melting temperature between the overlap portions 112 and 122, Tm2 denotes the melting temperature between the head oligo targeting portion 111 and the first target region 21 on the the target nucleic acid 2, and Tm3 denotes the melting temperature between the tail oligo targeting portion 123 and the second target region 22 on the the target nucleic acid 2. In Figure 1B, the reporter probe 1 depicted in Figure 1A is shown bound to a target nucleic acid 2. Both the head oligonucleotide 11 and the tail oligonucleotide 12 of the reporter probe 1 are bound in close proximity to each other to the target regions 21, 22 on the target nucleic acid 2 with their respective targeting portions 111, 123. The overlap portion 112 at the 3' end of the head oligo 11 anneals to the overlap portion 122 of the tail oligo 12, forming a double helix. The overlap portions 112, 122 are configured to be able to specifically anneal to each other, but not to the target nucleic acid 2. Consequently, in its bound state, the overlap portions 112, 122 and the labeling portion 121 of the tail oligo 12 form a branch 126, branching off from the target nucleic acid 2.

Figure 2 shows the embodiment of the reporter probe 1 of the invention shown in Figure 1. Nucleobases are symbolized as crossbars extending from the sugar-phosphate backbone symbolized as a line. Figur 2A shows the configuration of Fig. 1B, i.e., the reporter probe 1 reversibly bound via Watson-Crick base pairing to the target regions 21, 222 on the target nucleic acid 2. Fig. 1A depicts a status, in which the labeling portion 121 of the tail oligo 12 is in a single-stranded state. In this state, the fluorescence of the flurorophore 125 is quenched by the quencher 124. Figure 2B schematically shows a polymerase 3 extending the 3'-end of the head oligo in 5'-3' direction taking the single-stranded labeling portion 121, i.e., the third nucleotide sequence, of the tail oligo 12 as a template, synthesizing a strand 113 complementary to the third nucleotide sequence 121, thus forming a double-stranded labeling portion 121. In this state, the fluorophore 125 is no longer quenched by the quencher 124 and fluoresces.

Figure 3 shows schematically and in a simplified manner a use of a reporter probe 1 of the invention in the context of a PCR method. A primer 4 (only the forward primer is shown here, Fig. 3A) anneals to the respective region on the target nucleic acid 2 to be amplified. A polymerase 3 having either exonuclease activity or strand displacement activity extends the 3'-end of the primer 4 and synthesizes a new strand 200 complementary to the template strand of the target nucleic acid 2, thereby degrading, in case the polymerase 3 has exonuclease activity, the parts of the reporter probe 1 binding, via Watson-Crick base pairing, to the target nucleic acid 2 (Fig. 3B). Degradation is symbolized here by fragments 114, 127 of the first and second oligonucleotides 11, 12 of the reporter probe 1. The double-stranded part of the reporter probe 1 branching from the target nucleic acid 2 is released. It fluoresces as long as it is in the double-stranded state. At a melting temperature Tm4, the double-stranded reporter part, comprising the overlap portions 112, 122 and the double-stranded labeling portion comprised of the third nucleotide sequence 121 and the strand 113 complementary thereto, melt, and the quencher 124 quenches the fluorescence of the florophore 125 (Fig. 3B). Tm4 is preferably equal to or higher than Tm2 and Tm3. A melting curve analysis can, for example, be performed measuring fluorescence of the reporter probe 1 of the invention. Unreacted (and thus still single-stranded) tail oligos do not show fluorescence. Without binding to a target nucleic acid 2 the head oligo 11 and tail oligo 12 do not bind to each other, at least not at temperatures above Tm1, and no double-strand of the labeling portion 121 will form.

Figure 4 schematically shows the four possible states of the reporter probe as the result of an assay in which a polymerase having exonuclease activity has been use. Figure 4A shows the reacted probe at a temperature below Tm4. The duplex between the tail oligo 12, here comprising the remaining labeling portion 121 and the overlap portion 122, and the head oligo 11 here composed of the overlap portion 112 and the newly synthesized complementary strand 113 to the labeling portion 121, is intact at a temperature below the melting temperature Tm4 of the duplex, and fluorescence light is emitted. The dublex has been formed as a result of the reaction of the reporter probe 1 with the target nucleic acid 2, i.e. the binding to the target nucleic acid 2. Figure 4B shows the state of a reacted probe at a temperature above Tm4. The duplex is melted and the two strands of the duplex are not annealed to each other. No fluorescence light is emitted because the fluorophore 125 and quencher 124 are in close proximity to each other, such that the quencher 124 quenches the fluorescence of the fluorophore 125. Figure C and D show the state of an unreacted reporter probe 1 at a temperature below (C) and above (D) Tm4. The reporter probe 1 has not been degraded, i.e. still comprises both targeting portions 111, 123, because the reporter probe 1 did not bind to a target nucleic acid 2. Head oligo 11 and tail oligo 12 do not anneal to each other when the temperature is above Tm1. No fluorescence light is emitted. Also at a temperature above Tm4, the configuration of the reporter probe (separted oligs; no duplex formation) is the same, and no fluorescence is observed.

Figure 5 shows in a simplified manner steps of a PCR method of the invention (TBOE PCR) using an embodiment of a reporter probe 1 of the invention, and the result of a melting curve analysis using an embodiment of a reporter probe of the invention 1. Fig. 5A-C show schematically and simplified the annealing of an embodiment of a reporter probe 1 of the invention to a target nucleic acid 2. Two primers 4, a forward and a reverse primer, also anneal to the target nucleic acid 2. A polymerase 3 (not shown here, and only representated as doted arrows) amplify the target nucleic acid 2, and form the complementary strand 113 (see Fig. 5C) of the labeling portion 121 of the reporter probe 1. Not shown in the figure is that the annealing step for the reporter probe 1 is preferably performed at a first annealing temperature (e.g. 68 °C) at which no or only a small fraction of the primers 4 will bind, favoring the formation of the complementary strand 113 by the polymerase 3. For the binding of the primers 4, the temperature is subsequently preferably lowered to a lower second annealing temperature (e.g. 60 °C). In the embodiment of a PCR method of the invention depicted here the targeting portions 111, 123 of the reporter probe 1 are degraded during the PCR. In other embodiments, for example in an embodiment of the method of the invention configured as an isothermal nucleic acid amplification method (e.g. LAMP), the reporter probe 1 may be simply displaced by a polymerase without 5' exonuclease activity and released from the target nucleic acid 2 without cleavage. Shown in Fig. 5D is the negative first derivative of the fluorescence (-dF/dT) in relation to temperature (T). A melting curve analysis may, for example, be performed after PCR or any isothermal amplification (e.g. LAMP) in order to determine the specificity of the amplicons obtained during amplification. The temperature of the reaction mixture containing the amplicons is raised and the fluorescence is measured. As schematically depicted in Figure 5, the embodiment of the reporter probe 1 of the invention used here has been degraded, e.g. by a polymerase having exonuclease activity, such that only the double-stranded branch 126 comprising the overlapping portions 112, 122 of the head and tail oligos 11, 12, the labeling portion 121 of the tail oligo 12 and its complementary strand 113 synthesized, e.g., during amplification, are left. The dual labled reporter probe 1, or the remaining double-stranded branch 126, fluoresces when in double-stranded state (left from peak). Fluorescence is, however, quenched when the reporter probe 1, or the remaining rest thereof, melts, i.e., when the two strands of the branch 126 separate (right from peak). Fluorescence thus decreases with increasing dissociation of the double-stranded probe 1.

Figure 6 shows simplified a representation of an embodiment of a reporter probe 1 of the invention bound to a part of a BlaGES-5 gene (Klebsielle pneumoniae, GenBank: DQ902344.1). Reference signs correspond to those mentioned above. Sequences of the double strand of the part of the BlaGES gene shown are given in SEQ ID NO: 35 and SEQ ID NO: 36. Sequences of the first oligonucleotide (head oligonucleotide) 11 and the second oligonucleotide (tail oligonucleotide) 12 are given in SEQ ID NO: 8 and SEQ ID NO: 9, respectively.

### EXAMPLES

In the following, the invention will be described for illustration purposes by way of examples for the use of the reporter probe of the invention and methods using the reporter probe of the invention in molecular infection diagnostics, in particular in the context of pathogenic bacteria that are resistant against β-lactam antibiotics. A PCR method and a LAMP method using the reporter probe of the invention will be described in more detail.

Resistance to β-lactam antibiotics based on the acquisition of genes that encode β-lactamases (bla genes) poses serious problems for the treatment of patients infected with Gram-negative pathogenic bacteria (e.g., Enterobacteriaceae, Pseudomonas spp., and Acinetobacter baumannii). Important examples of β-lactamases are extended-spectrum β-lactamases (ESBLs) and carbapenemases. An example of ESBLs are Guiana extended-spectrum (GES) β-lactamases (BlaGES), variants of which, however, also carbapenemase activity (Castanheira M, Simner PJ, Bradford PA. Extended-spectrum β-lactamases: an update on their characteristics, epidemiology and detection. JAC Antimicrob Resist. 2021 Jul 16;3(3):dlab092. doi: 10.1093/jacamr/dlab092). The enzyme BlaGES-5, for example, has carbapenemase activitity (Ota Y, Prah I, Mahazu S, Gu Y, Nukui Y, Koike R and Saito R (2023) Novel insights into genetic characteristics of blaGES-encoding plasmids from hospital sewage. Front. Microbiol. 14:1209195. doi: 10.3389/fmicb.2023.1209195).

In the following, a method using the reporter probe of the invention will be designated "TBOE"-Assay, in order to distinguish the invention from prior art methods.

### Example 1: TBOE signals are temperature dependent and do not require 5' exonuclease activity

The beta-lactamase and carbapenemase gene "blaGES" is used as an exemplary target for this experiment.

In order to verify and compare the TBOE reporter concept with established qPCR reporter concepts, three assay variants for detection of blaGES featuring a Taqman reporter, a TOCE reporter or a TBOE reporter, respectively, were combined with one mastermix containing a Taq-polymerase (Quanta perfeCTa), and a second mastermix containing a proofreading polymerase instead (Quanta repliQa). Both versions of each assay were tested on a 10-fold dilution series of a blaGES positive clinical isolate, on a CFX96 Instrument (Bio-rad).

Oligos used for this experiment (5' → 3' direction)

| | |
|---|---|
| GES-fwd | CTGTGGCTAAAGTCCTCTATG (SEQ ID NO: 1) |
| GES-rev | CAACAACCCAATCTTTAGGAAA (SEQ ID NO: 2) |
| GES-Probe | FAM- CGT CTC CCG (ZEN)TTT GGT TTC CG - IBFQ (SEQ ID NO: 3) |
| AR1-GES-tc1-P1 | GGT CCT TCA TCG CTC CGT CTC CCG TTT GGT TTC CG -C3- Spacer (SEQ ID NO: 4) |
| AR1-GES-tc1-C1 | 5'BHQ2- TTT TTT TTT TTT TTT TC(FAM-dT) CTC TTG ACG GAG CGA TGA AGG ACC -C3-Spacer (SEQ ID NO: 5) |
| v3GES-Fv2 | AACACACCTGGCGACCT (SEQ ID NO: 6) |
| v3GES-Rv4 | CAATCTTTAGGAAAACCCGC (SEQ ID NO: 7) |
| v3GES-H1-a | GTCGCGTCTCCCGTTTGGTTT T AT GAC TCT (SEQ ID NO: 8) |
| v3GES-T1-a | 5'BHQ2- AAA CAG CCC GCG GTC GTT T(TexasRed-dT)C CTT TT AGA GTC AT T CGATCAGCCACCTCTCAATGGTGTGGGT -C3- Spacer (SEQ ID NO: 9) |

ZEN (Integrated DNA Technologies, IDT; see, e.g., US 7439341 B2), IBFQ (Iowa Black FQ quencher, IDT): quenchers
Run-protocols used for this experiment (CFX96)
Quanta_perfeCTa_TBOE_generic

| Step | Temp. | Duration [min:s] | Operation |
|---|---|---|---|
| 1 | 95°C | 5:00 | Hold |
| 2 | 95°C | 0:10 | Cycle |
| 3 4 | 68°C | 0:05 | |
| | 60°C | 0:25 | |
| | | | +Detect |
| 5 | 80°C | 0:05 | |
| | | | +Detect |
| | go to Step 2: 39x | | |
| 6 | 58°C | 0:30 | Hold |
| 7 | 85°C | continuous | Melt |

Quanta_repliQa_TBOE

| Step | Temp. | Duration [min:s] | Operation |
|---|---|---|---|
| 1 | 98°C | 0:30 | Hold |
| 2 | 98°C | 0:10 | Cycle |
| 3 | 68°C | 0:05 | |
| 4 | 60°C | 0:20 | |
| | | | +Detect |
| 5 | 80°C | 0:05 | |
| | | | +Detect |
| | go to Step 2: 39x | | |
| 6 | 58°C | 0:30 | Hold |
| 7 | 85°C | continuous | Melt |

Using the Taq-polymerase mastermix (figure 7a) all three assays yield detectable signals during amplification. Taq-polymerase has 5' exonucleasae activity. For the TOCE (middle) and TBOE assays (right), a melt-curve is detectable after amplification, whereas the Taqman assay did not generate a melt-curve.

Using the HiFi-polymerase mastermix (figure 7b) comprising a polymerase without the 5' exonucleasae activity, the Taqman reactions expectedly yield only very weak and abnormal amplification signals, whereas TOCE does not generate any signals at all. In contrast the TBOE assay generated strong signals and melt-curves, similar to what was observed in Taqman mastermix.

### Example 2: TBOE can be used with loop-mediated amplification (LAMP) assays

To demonstrate the functionality of a TBOE reporter of the invention in conjunction with isothermal amplification methods, a primer set for LAMP reactions, again targeting the blaGES gene, was created in such a way to incorporate the TBOE reporter system in one of the loop regions (similar to a loop primer, see figure 8). The assay was compared to the same primer set with high-resolution dye (ResoLight, Roche) using a 10-fold dilution series similar to example 1 (Figure 9).

Oligos used for this experiment (5' → 3' direction)

| | |
|---|---|
| v3GES-L-F2L | TTAGGAAAACCCGCTCGTA TTT GCGCACTGACGTCCAC (SEQ ID NO: 10) |
| v3GES-L-F3 | CGTACTGTGGCTAAAGTCCT (SEQ ID NO: 11) |
| v3GES-L-R2L | CCTGCGCCAACGGG TTT GTAATCTCTCTCCTGGGCTT (SEQ ID NO: 12) |
| v3GES-L-R3 | GCCGTTGTATACACCGCT (SEQ ID NO: 13) |
| v3GES-L-RLP | CCGGAACGACATTGGTTT (SEQ ID NO: 14) |
| v3GES-H1-a | GTCGCGTCTCCCGTTTGGTTT T AT GAC TCT (SEQ ID NO: 15) |
| v3GES-T1-a | 5'BHQ2- AAA CAG CCC GCG GTC GTT T(TexasRed-dT)C CTT TT AGA GTC AT T CGATCAGCCACCTCTCAATGGTGTGGGT -C3- Spacer (SEQ ID NO: 16) |

Run-protocols used for this experiment (CFX96)

### NEB WarmStart TBOE

| Step | Temp. | Duration [min:s] | Operation |
|---|---|---|---|
| 1 | 65°C | 0:30 | Hold |
| 2 | 60°C | 0:30 | Cycle |
| | | | +Detect |
| | go to Step 2: 49x | | |
| 3 | 95°C | 0:30 | |
| 4 | 55°C | 0:30 | Hold |
| 5 | 85°C | continuous | Melt |

The double strand dye version of the assay generated strong signals during amplification but no discernible melt-peaks. The TBOE-LAMP assay generated strong signals and melt-curves detectable after amplification (figure 9).

### Example 3: TBOE used for temperature-dependent multiplexing

A set of assays for blaVIM, blaNDM and blaOXA-48-like was used to demonstrate assay differentiation through melt curve analysis and different temperature levels during qPCR amplification. blaOXA-48 and blaNDM assays were designed to be undetectable at 72°C and 90°C respectively. For blaVIM, a Taqman assay was used (not temperature dependent). The assays were run on every possible combination of targets in triplicates to verify functionality (Figures 10-11).

Oligos used for this experiment (5' → 3' direction)

| | |
|---|---|
| OXA48-Fwd | AGGGCGTAGTTGTGC(O-meth-U)C (SEQ ID NO: 17) |
| OXA48-Rev | GTGTTCATCCTTAACCAC(O-meth-G)C (SEQ ID NO: 18) |
| NDM-Fwd | GCCACACCAGTGACAATA(O-meth-U)C (SEQ ID NO: 19) |
| NDM-Rev | GTGCTCAGTGTCGG(O-meth-C)AT (SEQ ID NO: 20) |
| v3VIM-F1 | CCR TCC AAT GGT CTC ATT GTC C (SEQ ID NO: 21) |
| v3VIM-F2 | TCG TCT AAT GGR CTT ATC GTC C (SEQ ID NO: 22) |
| v3VIM-R1 | GCC GAC GCG GTC GTC AT (SEQ ID NO: 23) |
| v3VIM-R2 | CCA CCG ACT CGA TCG TCA T (SEQ ID NO: 24) |
| Oxa48-be1-head1 | GAA TAA GCA GCA AGG ATT TAC CAA TAA TCT T  TTT CCT ACG (SEQ ID NO: 25) |
| Oxa48-be1-tail1 | 5'BHQ2- CGT TCC GTC TTT TTT TTT TC(JOE-dT) TCT T CGT AGG AAT AAC GGG CGA ACC AAG CAT TTT TAC C -C3-Spacer (SEQ ID NO: 26) |
| NDM-be2-head1 | GCG ACT TGG CCT TGC TGT AAA CCT GAG (SEQ ID NO: 27) |
| NDM-be2-tail1 | 5'BHQ2- GCC GTG CTC GTC GTC CTC CT(JOE-dT) CTC T CTC AGG TTA CTT GAT CAG GCA GCC ACC AAA AGC G -C3-Spacer (SEQ ID NO: 28) |
| VIM-P | SUN- ATGAGTTGC(ZEN)TTTTGATTGATACAGCKTGG -IBFQ (SEQ ID NO: 29) |

Run-protocols used for this experiment (CFX96)

| Step | Temp. | Duration [min:s] | Operation |
|---|---|---|---|
| 1 | 95°C | 5:00 | Hold |
| 2 | 95°C | 0:10 | Cycle |
| 3 | 68°C | 0:05 | |
| 4 | 60°C | 0:25 | |
| | | | +Detect |
| 5 | 72°C | 0:05 | |
| | | | +Detect |
| 6 | 90°C | 0:05 | |
| | | | +Detect |
| | go to Step 2: 39x | | |
| 7 | 58°C | 0:30 | Hold |
| 8 | 85°C | continuous | Melt |

For differentiation by melt-curve analysis, each peak above threshold constituted a positive result for the allocated target. All samples and negative control were correctly detected by the multiplex test with amplification curves and melt curves in the correct optical channel, only counting the TBOE-based targets (Figure 10). The TaqMan assay (VIM) did not generate a melt curve.

For differentiation by detection at different temperature levels, in a first step, amplification signals are detected during amplification at 60°C (step 4), 72°C (step 5) and 90°C (step 6) (figure 11a). Target specific signals were extracted from the raw data by compensating between the different steps as shown in figure 11. Compensated signals are unambiguous for the respective targets and a deconflicted table of end-point fluorescence showed a perfect match to the experiment setup (figure 11).

### Example 4: Detection of single nucleotide polymorphisms with TBOE

In order to demonstrate the possibility to discriminate single-nucleotide polymorphisms (SNP) using a TBOE assay, a Neisseria gonorrhoeae gyrA Ser91Phe (Quinolone resistance marker) assay was created, using a set of two competitive TBOE tails, one for each sequence variation. LNA was used to increase sequence specificity for the tail-oligos target regions.

Oligos used for this experiment (5' → 3' direction)

| | |
|---|---|
| NG-gyra-91-F | CTGGAATGCCGCCTACAA (SEQ ID NO: 30) |
| NG-gyra-91-R | GTTGCCCTGTCCGTCTATC (SEQ ID NO: 31) |
| NG-gyra-91-H1-b | CGGTAAATACCACCCCCACGGC T TA TGC TCT A (SEQ ID NO: 32) |
| NG-gyra-91ser-T1-a | BHQ2- AAA GTG CCG GTC TTT TTT C(JOE-dT)T TTC TT AGA GCA TA T ATT +CCG CAG TTT ACG VCA CCA T -C3-Spacer (SEQ ID NO: 33) |
| NG-gyra-91phe-T1-a | BHQ2- AAA CTC GCC GCG GTC CTC C(JOE-dT)T TTT CT AGA GCA TA T ATT +TCG CAG TTT ACG VCA CCA TC -C3-Spacer (SEQ ID NO: 34) |

"+C" and "+T" stand for the respective LNA nucleotides (LNA cytosine and LNA thymine). "C3-Spacer" stands vor a 3' C3 (C₃H₃O₄P) spacer.

Run-protocols used for this experiment (CFX96)

| Step | Temp. | Duration [min:s] | Operation |
|---|---|---|---|
| 1 | 95°C | 5:00 | Hold |
| 2 | 95°C | 0:10 | Cycle |
| 3 | 68°C | 0:05 | |
| 4 | 60°C | 0:25 | |
| | | | +Detect |
| 5 | 80°C | 0:05 | |
| | | | +Detect |
| | go to St ep 2: 39x | | |
| 6 | 58°C | 0:30 | Hold |
| 7 | 85°C | continuous | Melt |

To distinguish the two relevant sequence variations using the TBOE assay, melt curve analysis was carried out, whereby a maximum peak at approximately 66.5°C indicated the wild type sequence, and a maximum peak at approximately 72°C indicated the mutated sequence for Ser91Phe (Quinolone resistant) (see figure 12). Two clinical isolates, predermined with the Seegene Allplex NG + DR were used to demonstrate the capability to differentiate between the two sequence variant.

Other sequences:
Sequence of part of BlaGES-5 gene in Fig. 6: SEQ ID NO: 35
Sequence of reverse complement of part of BlaGES-5 gene in Fig. 6: SEQ ID NO: 36

## Claims

1. A reporter probe for the detection of a target nucleic acid, the reporter probe comprising
i) a first oligonucleotide, the first oligonucleotide comprising, consecutively in 5'-3' direction, a) a first nucleotide sequence complementary to a first target region of the target nucleic acid and b) a second nucleotide sequence not complementary to the target nucleic acid, and
ii) a second oligonucleotide, the second oligonucleotide comprising, consecutively in 5'-3' direction, c) a third nucleotide sequence comprising a fluorophore-quencher pair, d) a fourth nucleotide sequence not complementary to the target nucleic acid but reverse complemantary to the second nucleotide sequence of the first oligonucleotide, and e) a fifth nucleotide sequence complementary to a second target region of the target nucleic acid, the second target region being adjacent to the first target region of the target nucleic acid.

2. The reporter probe according to claim 1, wherein the fluorophore-quencher pair is arranged in the third nucleotide sequence of the second oligonucleotide in such a manner that the fluorescence of the fluorophore is quenched in a single-stranded state of the third nucleotide sequence.

3. The reporter probe according to one of claims 1 or 2, wherein the quencher, preferably a dark quencher, is arranged at or near the 5' end of the third nucleotide sequence of the second oligonucleotide, and wherein the fluorophore is preferably arranged at or near the 3' end of the the third nucleotide sequence.

4. The reporter probe according to one of the preceding claims, wherein the first oligonucleotide is shorter in length than the second oligonucleotide.

5. The reporter probe according to one of the preceding claims, wherein a first melting temperature, Tm₁, of a duplex formed between the second and the fourth nucleotide sequences of the first and second oligonucleotides of the reporter probe is lower than a second and third melting temperature, Tm₂, Tm₃, of a duplex formed between either the first or the fifth nucleotide sequence of the first and second oligonucleotides of the reporter probe with the target nucleic acid.

6. The reporter probe according to claim 5, wherein Tm₁ is in the range of 10-20 °C, preferably 12-18 °C, more preferably 14-17 °C, and Tm₂ and Tm₃ each are in the range of 60-80 °C, preferably 60-75 °C, 62-73 °C, 64-72 °C, 65-70 °C or 65-68 °C.

7. The reporter probe according to one of claims 5 or 6, wherein a fourth melting temperature, Tm4, for a duplex formed between the first and second oligonucleotide after extension of the 3'-end of the second nucleotide sequence and formation of a double-stranded third nucleotide sequence is preferably equal to or higher than Tm2 and Tm3.

8. A method for the detection of a target nucleic acid, comprising the steps of
a) contacting the target nucleic acid with a reporter probe of one of claims 1 to 7 targeting the target nucleic acid, in the presence of a polymerase and nucleoside triphosphates, dNTPs, under conditions to allow nucleic acid synthesis by the polymerase, and
b) detecting the fluorescence of the reporter probe.

9. The method of claim 8, further comprising the step of
- amplifying the target nucleic acid before or during step b) of detecting the fluorescence of the reporter probe.

10. The method according to claim 9, wherein the target nucleic acid is, in step a), contacted with at least one primer pair, preferably a primer pair comprising a forward and reverse primer.

11. A kit for the detection of a target nucleic acid, the kit comprising a reporter probe of one of claims 1 to 7.

12. The kit of claim 11, further comprising a polymerase, preferably a heat-stable polymerase and nucleoside triphosphates, dNTPs.

13. The kit of one of claims 11 or 12, further comprising at least one pair of primers configured to bind to the target nucleic acid.

14. Use of a reporter probe of one of claims 1 to 7 for the detection of a target nucleic acid in a biological sample.

15. Use according to claim 14, wherein the detection of the target nucleic acid includes a step of amplifying the target nucleic acid.
